(19) Europäisches Patentamt
European Patent Office
Office européen des brevets

(11) **EP 4 710 953 A1**

(12) **EUROPÄISCHE PATENTANMELDUNG**

(43) Veröffentlichungstag:
**18.03.2026 Patentblatt 2026/12**

(21) Anmeldenummer: 24315419.2

(22) Anmeldetag: **16.09.2024**

(51) Internationale Patentklassifikation (IPC):
*A61L 15/22* (2006.01)   *A61L 15/46* (2006.01)
*A61L 15/60* (2006.01)

(52) Gemeinsame Patentklassifikation (CPC):
(C-Sets verfügbar)
**A61L 15/46; A61L 15/225; A61L 15/60;**
A61L 2300/252; A61L 2300/404         (Forts.)

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Paul Hartmann AG**
**89522 Heidenheim (DE)**

(72) Erfinder:
• **KETTEL, Markus**
**89522 Heidenheim (DE)**

• **KARL, Michael Maximilian**
**89522 Heidenheim (DE)**
• **MERCKEL, Fabien**
**67100 Strasbourg (FR)**
• **VRANA, Nihal Engin**
**67100 Strasbourg (FR)**
• **HATHROUBI, Skander**
**67100 Strasbourg (FR)**

(74) Vertreter: **Paul Hartmann AG**
**Patents & Licensing**
**Paul-Hartmann-Straße 12**
**89522 Heidenheim (DE)**

(54) **VLIESSTOFF MIT INFEKTIONSHEMMENDEN EIGENSCHAFTEN**

(57)    Die vorliegende Erfindung betrifft einen Vliesstoff zur Wundbehandlung, welcher teilweise oder vollständig eine antimikrobielle Beschichtung aufweist. Die Beschichtung umfasst eine Hyaluronsäure und ein Polypeptid ausgewählt aus Polyarginin, Polylysin und Polyornithin oder einer Mischung aus mindestens zwei der zuvor genannten Polypeptide. Darüber hinaus werden Verfahren zum Auftragen der Beschichtung auf den Vliesstoff sowie Wundauflagen umfassend den antimikrobiellen Vliesstoff beschrieben. Die Beschichtung zeichnet sich durch ihre gute Verträglichkeit und Zellkompatibilität bei gleichzeitig starker antiseptischer Wirkung aus.

Figur 3b

EP 4 710 953 A1

(52) Gemeinsame Patentklassifikation (CPC): (Forts.)

C-Sets
**A61L 15/225, C08L 5/08;**
**A61L 15/225, C08L 77/04**

## Beschreibung

### Technisches Gebiet der Erfindung

Die vorliegende Erfindung betrifft die Wundbehandlung, insbesondere die Behandlung schwerer, chronischer und/oder infizierter Wunden. Spezifische Ausführungsformen der Erfindung ermöglichen zudem die Behandlung stark exsudierender (nässender) Wunden.

### Hintergrund der Erfindung

Chronische Wunden stellen in der modernen Medizin nach wie vor ein Problem dar. Gerade bei älteren Menschen und Risikopatienten wie beispielsweise Diabetikern besteht ein erhöhtes Risiko, dass Gewebeschäden nicht oder nicht vollständig abheilen. Der Wundheilungsprozess ist in einem solchen Fall aus unterschiedlichen Gründen gestört und es besteht ein fortwährender Defekt der Hautbarriere. Dies geht zum einen mit einer Reduktion der Lebensqualität für die Betroffenen einher. Zum anderen erhöht sich mit fortwährender Dauer der Gewebeexposition zunehmend das Risiko einer Infektion. Tritt eine solche Infektion ein, verschlechtert sich die Prognose weiter. Da die körpereigenen Stoffwechselprozesse im Bereich der chronischen Wunde beeinträchtigt sind und eine reguläre Immunantwort nicht oder nicht vollständig erfolgt, kann es in der Folge zu einer Vermehrung der Erreger an der Infektionsstelle kommen. Im weiteren Verlauf bildet sich häufig ein Biofilm, bei dem bakterielle Erreger sich zu einer Lebensgemeinschaft vereinen, die erhöhte Resistenz gegenüber Bioziden und Antibiotika zeigt und daher die weitere Behandlung außerordentlich erschwert. Dieses Stadium erhöht die Wahrscheinlichkeit für weitere Komplikationen wie beispielsweise Nekrosenbildung oder Sepsis.

Aus dem Stand der Technik sind Wundauflagen mit antibiotischer Wirksamkeit bekannt. Diese beruhen in der Regel auf der Verwendung körperfremder antimikrobieller Wirkstoffe. Derartige Wundauflagen zeigen zwar eine Wirkung gegen pathogene Mikroorganismen, haben jedoch die nachteilige Eigenschaft auch körpereigene Zellen zu beeinträchtigen. Mittels *in vitro* Assays konnte eine erhöhte Zytotoxizität für tierische Zellen nachgewiesen werden. Die betroffenen Zellen werden gestresst und ihre Vitalwerte sinken ab. Bei Testkonzentrationen, die den Realbedingungen nahekommen, stirbt für gewöhnlich ein Teil der im Assay befindlichen Zellen ab.

Die EP 1 755 569 B9 beschreibt eine Wundauflage mit Salbe, die zusätzlich ein antibakterielles Metall wie beispielsweise Silber enthält. Allerdings geht die Wirkung von Silber mit deutlich messbarer Zytotoxizität einher.

Die EP 3 452 118 B1 beschreibt antimikrobielle Beschichtungen, die Hyaluronsäure und ein Polypeptid enthalten, wobei die Säure und das Peptid nicht durchmischt vorliegen. Spezifische Ausgestaltungen von Wundauflagen werden nicht genannt.

Die EP 2 371 335 B1 beschreibt eine antibakterielle Wundauflage mit dem Wirkstoff Polyhexamethylenbiguanid (PHMB). Jüngste Untersuchungen haben jedoch gezeigt, dass PHMB problematischer ist, als ursprünglich angenommen wurde. So wurden toxische Effekt im *in vitro* Assay bei Humanzellen festgestellt (Medical mycology, 2017, 55. Jg., Nr. 3, S. 334-343) und auch *in vivo* Versuche an Ratten zeigten ein ernstzunehmendes Schädigungspotential (Interdisciplinary Toxicology, 2015, 8. Jg., Nr. 4, S. 193-202).

Folglich besteht ein Bedarf nach einem spezifisch ausgestalteten Mittel zur Wundabdeckung, das ausreichende antimikrobielle Wirksamkeit zeigt, nicht cytotoxisch für menschliche Zellen ist und zudem atraumatisch und schmerzfrei entfernt werden kann. Um dem klinischen Alltag gerecht zu werden, sollte das Mittel der Wahl zudem unmittelbar einsetzbar sein, bei längerer Lagerung über eine ausreichende Stabilität verfügen und beständig gegenüber möglichen Temperaturschwankungen sein.

### Zusammenfassung der Erfindung

Die oben genannte Aufgabe wird gelöst durch einen Vliesstoff zur Wundbehandlung, welcher teilweise oder vollständig eine antimikrobielle Beschichtung aufweist, welche i) eine Hyaluronsäure sowie ii) ein Polypeptid ausgewählt aus Polyarginin, Polylysin und Polyornithin oder eine Mischung aus mindestens zwei der zuvor genannten Polypeptide umfasst.

Der erfindungsgemäße Vliesstoff hat hervorragende atraumatische Eigenschaften, d.h. er verbindet sich nicht mit der Wunde oder Wundbestandteilen. Weder kann Gewebe in den Vliesstoff einwachsen, noch kann der Vliesstoff am Wundbett ankleben. Der für gewöhnlich notwendige Einsatz von Salbe oder Silikongel zum Erreichen der atraumatischen Eigenschaften ist bei der vorliegenden Erfindung nicht notwendig, da diese Rolle von der antimikrobiellen Beschichtung erfüllt wird. Schließlich ist die Beschichtung aufgrund ihrer antimikrobiellen Inhaltsstoffe gegen humanpathogene Bakterien wirksam. Somit werden durch die Beschichtung gleich zwei vorteilhafte Wirkungen erzielt. Zudem kann der beschichtete Vliesstoff als Teil von Wundauflagen dienen. Die Wundauflage kann dabei für die Behandlung unterschiedlicher Wundarten spezifisch zusammengestellt werden, um die bestmögliche Versorgung zu gewährleisten.

Der erfindungsgemäße Vliesstoff kann auch dann zum Einsatz kommen, wenn sich in der zu behandelnden Wunde antibiotikaresistente Bakterien befinden. Während Antibiotika üblicherweise organische Verbindungen sind, die von resistenten Bakterien beispielsweise abgebaut werden, ist ein derartiges Auftreten von Resistenzen im Hinblick auf die antimikrobielle Beschichtung der vorliegenden Erfindung nicht beobachtet worden.

Weiterhin kann der erfindungsgemäße Vliesstoff wundheilungsfördernde Eigenschaften aufweisen. Dies ergibt sich durch die feuchtigkeitsregulierenden Eigenschaften der Hyaluronsäure, die auch *in vivo* im Bereich der extrazellulären Matrix zu finden ist.

Nachfolgend wird erläutert, wie der Vliesstoff und die zugehörige Beschichtung strukturell und chemisch beschaffen sein können, um in der Praxis den größtmöglichen Nutzen zu erbringen.

**Detaillierte Beschreibung der Erfindung**

Der Begriff "medizinisch akzeptables Material" im Sinne der Erfindung ist eine ungiftige, fusselfreie und stabile Substanz (z.B. ein Substrat), die sich unter Normalbedingungen weder in polaren noch in unpolaren Verbindungen löst und weder von den Absonderungen tierischer noch bakterieller Zellen in nennenswertem Ausmaß abgebaut oder verflüssigt werden kann.

Der Begriff "Vliesstoff" meint eine Schicht von miteinander zusammenhängenden Fasern, die nicht gewebt sind und in der Regel nicht nach einem sich wiederholendem Muster angeordnet sind.

Der Ausdruck "Kolonie bildende Einheit" (CFU) meint eine einzelne teilungsfähige Zelle eines Einzellers, insbesondere eines humanpathogenen Einzellers oder Bakteriums.

"Beschichtet" meint, dass eine Oberfläche eines Festkörpers (z.B. eines Vliesstoffes) mittels einer Substanz, die sich von der Struktur des Festkörpers unterscheidet, zumindest teilweise bedeckt bzw. überlagert wird. Insbesondere kann die Beschichtung faserlos und / oder gelartig sein. Somit kann es sich bei der Beschichtung um ein Gel, insbesondere ein hydrophiles Gel handeln.

Der Ausdruck "konfiguriert zum Auflegen auf eine Wunde" meint, zum Auflegen auf eine Wunde zu einem therapeutischen Zweck im Sinne einer Wundbehandlung vorgesehen und geeignet.

Der Ausdruck "atraumatisch" meint, dass ein Wundversorgungsprodukt sich nicht mit der Wunde fest verbindet, also beispielsweise nicht in der Wunde eintrocknet oder mit dieser verwächst und dass eine schmerzfreie Entfernung des Produktes ohne Störung des Heilungsprozesses möglich ist.

Der Ausdruck "antimikrobielle Inhaltsstoffe" meint Hyaluronsäure und ein oder mehrere Polypeptide, bei denen es sich um Polyarginin und / oder Polylysin und / oder Polyornithin handelt.

Der Ausdruck "antimikrobiell" meint im weitesten Sinne, dass ein Wirkstoff, Wirkstoffgemisch oder ein damit behandelter Artikel (z.B. ein Vliesstoff oder eine Wundauflage) in der Lage ist die Vermehrung von Mikroorganismen zu hemmen oder aufzuhalten oder die Anzahl vitaler Mikroorganismen zu reduzieren. Im engeren Sinne ist gemeint, dass ein solcher Artikel in der Lage ist in einem Test nach ISO 20743:2021 die Anzahl der CFU des *Pseudomonas aeruginosa* Stammes mit der Hinterlegungsnummer ATTC 27853 und / oder des *Staphylococcus aureus* Stammes mit der Hinterlegungsnummer ATTC 25923 um mindestens drei Logarithmusstufen, bevorzugt um mindestens vier Logarithmusstufen, besonders bevorzugt um mindestens fünf Logarithmusstufen und ganz besonders bevorzugt um mindestens sechs Logarithmusstufen im Vergleich zu einem baugleichen Artikel ohne Wirkstoff zu reduzieren. Darüber hinaus schließt der Ausdruck "antimikrobiell" den Ausdruck "antibakteriell" mit ein.

Die Ausdrücke "Aminosäure" und "Aminosäuren" beziehen sich sofern nicht anders angegeben, auf die Verbindungen Arginin, Lysin und / oder Ornithin, welche allesamt zu den positiv geladenen Aminosäuren zählen. Dies schließt insbesondere die L-Enantiomere der genannten Aminosäuren ein.

Die Ausdrücke "Polypeptid" "antimikrobielles Polypeptid" und "Polyaminosäure" meinen vorliegend Polyarginin, Polylysin und / oder Polyornithin und beziehen sich auf Peptidverbindungen, die mindestens 11, bevorzugt mindestens 20, besonders bevorzugt mindestens 30 Untereinheiten in Form von verbundenen Aminosäuren umfassen. Im Rahmen der Erfindung können die Aminosäuren innerhalb eines solchen Polypeptids allesamt identisch sein (selbe Aminosäure). Alternativ können Mischungen aus zwei oder drei der zuvor genannten Aminosäuren innerhalb eines Polypeptids vorliegen. Alle derartigen Polypeptide haben eine positive Nettoladung. Dem Fachmann ist bekannt, dass sich für die Aminosäuren am C-terminalen sowie N-terminalen Ende naturgemäß strukturelle Unterschiede ergeben, da diese Positionen die jeweiligen Kettenenden darstellen.

Der Ausdruck "Polylysin" umfasst die stereochemische Variante $\alpha$-Poly-L-Lysin und / oder $\varepsilon$-Poly-L-Lysin.

Die Ausdrücke "abwechseln" bzw. "abwechselnd" sowie "alternierend" meinen, dass auf eine erste Lage enthaltend eine Hyaluronsäure eine zweite Lage enthaltend das Polypeptid oder die Polypeptide folgt, Eine optionale dritte Lage würde folglich wieder eine Hyaluronsäure enthalten. Auf diese Weise wechseln sich die Lagen in ihrer Nettoladung ab und bilden aufgrund ihrer Anziehungskräfte eine stabile Beschichtung aus. Alternativ kann in diesem Sinne natürlich auch auf eine erste Lage enthaltend das Polypeptid oder die Polypeptide eine zweite Lage enthaltend eine Hyaluronsäure usw. folgen.

Der Ausdruck "proximal" bedeutet, dass ein Element im Einsatz zur Wunde oder Haut hin ausgerichtet bzw. wundzugewandt ist.

Der Ausdruck "distal" bedeutet, dass ein Element im Einsatz von der Wunde oder Haut weg zeigt bzw. wundabgewandt ist.

Der Ausdruck "Wundauflage" meint im Sinne der Erfindung ein Produkt zur Abdeckung von Wunden, das über einen erfindungsgemäßen Vliesstoff und mindestens eine weitere Schicht verfügt, wobei sich die mindestens eine weitere Schicht von dem erfindungsgemäßen Vliesstoff unterscheidet.

Die vorliegende Erfindung betrifft einen Vliesstoff. Dieser Vliesstoff kann allein verwendet werden oder nach dem Anbringen auf eine Wunde bei Bedarf durch einen absorbierenden Sekundärverband überlagert werden oder mittels Sekundärverband, Klebefolie, Klebestreifen oder anderer Fixiermittel am Ort der Wunde befestigt werden. Bevorzugt ist der Vliesstoff jedoch Teil einer Wundauflage und besonders bevorzugt befindet sich der Vliesstoff proximal innerhalb der Wundauflage, wobei die Beschichtung des Vliesstoffes im Einsatz zur Wunde hin ausgerichtet ist und dabei als Wundkontaktschicht fungieren kann.

Vorteilhaft ist, dass der Vliesstoff mittels der enthaltenen Beschichtung atraumatisch ist und nicht mit der Wunde verklebt. Dies ermöglicht ein schmerzfreies und unkompliziertes Ablösen des Verbandsmaterials. Gleichzeitig erlaubt die Beschichtung eine leichte Initialhaftung, und zwar insbesondere an trockenem Gewebe. Dies erleichtert das Anbringen des beschichteten Vliesstoffs im Bereich der Wunde, denn in den meisten Fällen muss das Material nicht bis zur Applikation eines Fixiermittels an der Wundstelle festgehalten werden, sondern haftet zunächst von allein, so dass dem Anwender beide Hände für die Vorbereitung eines Fixiermittels (z.B. Klebestreifen oder Klebefolie) zur Verfügung stehen. Daneben ist auch die Verwendung des Vliesstoffes in Wundauflagen mit Klebekomponenten vorgesehen. Eine mögliche Ausgestaltung einer solchen Wundauflage ist der sogenannte Inselverband mit umlaufendem Kleberand. Dieser Kleberand kann mittels hautkompatibler Klebstoffe bereitgestellt werden, wobei Acrylkleber und Silikonkleber zu den gebräuchlichsten Mitteln gehören. Diese haben gleichzeitig den Vorteil restlos ablösbar zu sein.

Der erfindungsgemäße Vliesstoff eignet sich für akute (z.B. blutende) Wunden sowie für chronische Wunden. Insbesondere nässende (exsudierende) und / oder infizierte Wunden können hervorragend vorsorgt werden. Beispiele für mögliche exsudierende Wunden sind Dekubitus, Ulcus Cruris sowie exulzerierende Tumore. In der Praxis treten häufig Mischformen dieser Wunden auf, z.B. infizierte chronische Wunden. In solchen Fällen zeigen sich die vorteilhaften Eigenschaften des erfindungsgemäßen Vliesstoffes besonders deutlich.

Der erfindungsgemäße Vliesstoff umfasst eine antimikrobielle, insbesondere antibakterielle Beschichtung, die antimikrobielle Inhaltsstoffe enthält. Weitere Bestandteile können bei Bedarf hinzugefügt werden, um den Vliesstoff an den vorgesehenen Einsatzzweck anzupassen. Dies wird an anderer Stelle näher erläutert.

Im Rahmen der vorliegenden Erfindung kann vorgesehen sein, dass sich die Beschichtung ausschließlich auf der proximalen Seite des Vliesmaterials befindet und damit auf derjenigen Seite, welche in Benutzung der Wunde zugewandt ist.

Diese Ausführung hat den ökonomischen Vorteil, dass Beschichtungsmaterial gespart wird, ohne dass die Versorgung der Wunde darunter leidet.

Der Vliesstoff ist ein flexibler und elastischer Festkörper, der sich der Form der Körper- oder Wundoberfläche anpassen kann und Fasern enthält. Der Vliesstoff ist ein festes, unlösliches, medizinisch akzeptables Material. Das Material kann über eine Kristallgitterstruktur verfügen oder alternativ als teilkristalliner oder amorpher Festkörper vorliegen (z.B. als amorpher Thermoplast wie Polyvinylchlorid). Bei dem Material kann es sich um ein Polymer- oder um eine Polymermischung handeln, insbesondere aus einem thermoplastischen Polymer oder einer Mischung thermoplastischer Polymere.

In der Regel liegt der Vliesstoff als mindestens eine Schicht faserhaltigen Materials vor. Bevorzugt ist der Vliesstoff flach bzw. planar, so dass er eine im Wesentlichen einheitliche Dicke aufweist und weder die proximale noch die distale Seite nennenswerte (z.B. makroskopische) Unebenheiten oder Erhebungen aufweisen. Geringfügige produktionsbedingte Toleranzen sind hierbei zu vernachlässigen, so dass im Sinne der Erfindung eine einheitliche Dicke auch bei Abweichungen von +/-5 % als gegeben gelten soll. Ein flach ausgebildetes Vlies kann in der Draufsicht eine rechteckige, quadratische, ovale oder runde Form haben, wobei ovale oder runde Formen sich besonders für Wunden an Gelenken eignen und rechteckige oder quadratische Formen eine effizientere Ausbeute von Bahnenmaterial (z.B. mittels Schneiden oder Stanzen) ermöglichen und auch eine bessere Nutzung von Lagerplatz erlauben.

Daneben sind auch Ausgestaltungen in Form von Tamponaden - beispielsweise in Form eines Zylinders - möglich, um tiefe Wunde (Kavitäten) auszutamponieren. Für die Tamponade von Wunden sollte bevorzugt die gesamte (nach außen zeigende) Oberfläche des Vliesstoffes mit der antimikrobiellen Beschichtung versehen sein, um die Kontaktfläche der Beschichtung zum Gewebe zu maximieren.

Der Vliesstoff kann darüber hinaus als offene oder geschlossene Tasche vorliegen, also einen Innenraum aufweisen. Eine solche Tasche kann im Inneren mit

Füllmaterial bestückt sein. Das Füllmaterial kann beispielsweise Flocken (z.B. Zellstoffflocken) und / oder superabsorbierende Partikel umfassen. Bevorzugt ist das Füllmaterial saugfähig (absorbierend in Bezug auf polare Flüssigkeiten). Besonders bevorzugt ist das Füllmaterial saugfähig und hält aufgenommene Flüssigkeit nach der Absorption zurück, so dass es gleichzeitig retinierend ist.

Der beschichtete Vliesstoff kann Teil einer Wundauflage, insbesondere einer antimikrobiellen Wundauflage, sein. Der Umriss der gesamten Wundauflage kann mit dem Umriss des Vliesstoffes korrespondieren, was jedoch nicht zwingend erforderlich ist. Beispielsweise kann der Vliesstoff selbst rund sein und von einem rechteckigen umlaufenden Rand aus anderen Komponenten der Wundauflage umgeben sein. Ein solcher Rand kann aus demselben Material wie der Vliesstoff oder aus einem anderen Material bestehen. So kann der Rand durch eine Folie (z.B. aus Polyurethan) ausgebildet und optional auf der proximalen Seite mit einem Klebstoff beschichtet sein.

Der Vliesstoff oder seine Fasern können die folgenden Materialien oder Materialgemische enthalten oder daraus bestehen: Kunstfasern, Polyolefin basierte Fasern, Polyethylen, Polyetheretherketon (PEEK), Polyvinylchlorid (PVC), Polymethylmethacrylat (PMMA), Polykarbonat (PC), Polyester, Polyethylenterephthalat (PET), Polypropylen (PP), Polystyren (PS), Polyamid, Acrylnitril-Butadien-Styrol (ABS), Polylactide (PLA), Viskose, Zellulose basierte Fasern oder Mischungen aus zwei oder mehr der zuvor genannten Materialien. Bei Verwendung von Polyamid als Fasermaterial kann das Polyamid in Form von Nylon vorliegen. Mögliche Mischungen sind eine Mischung aus Polyamid und Viskose, eine Mischung aus Polypropylen und Viskose, eine Mischung von Polyethylen und Viskose, eine Mischung aus Polyester mit Baumwolle oder eine Mischung aus Polyester mit Viskose.

Synthetische Fasern wie beispielsweise Polyamid und Polyester haben den Vorteil, dass sie beständig sind gegen Zersetzung durch Mikroorganismen. Weiterhin haben viele dieser Fasern wie beispielsweise Polypropylen hervorragende thermoplastische Eigenschaften, was das Verarbeiten und Zusammenfügen von Schichten (z.B. mittels Schweißen) vereinfacht.

Der Vliesstoff enthält Fasern oder besteht ausschließlich aus Fasern. Bei den Fasern kann es sich um Fasern der oben genannten Materialien handeln, also zum Beispiel um Polyamidfasern, insbesondere Nylonfasern, Polyesterfasern, Polypropylenfasern, Baumwollfasern, Viskosefasern usw. Darüber hinaus können auch Mischfasern verwendet werden, welche eine Kombination aus zwei oder mehr der aufgeführten Materialien in einer Faser vereinen.

Möglich ist, dass der erfindungsgemäße Vliesstoff ausschließlich solche Fasern enthält, die synthetischen Ursprungs sind und / oder solche Fasern, die zu den Kunstfasern gezählt werden.

Der erfindungsgemäße Vliesstoff kann auch Regeneratfasern wie zum Beispiel Viskose enthalten.

Weiterhin ist auch möglich, dass der erfindungsgemäße Vliesstoff ausschließlich solche Fasern enthält, die natürlichen Ursprungs sind und / oder biologisch abbaubar sind. Bei der biologischen Abbaubarkeit kann es sich um eine Abbaubarkeit im Sinne der Norm EN ISO 14851 :2019 handeln. Fasern natürlichen Ursprungs sind beispielsweise Baumwollfasern. Fasern, die im Kontext der vorliegenden Erfindung als biologisch abbaubar gelten, sind beispielsweise Baumwollfasern und Viskosefasern. Im Gegensatz dazu sind Kunstfasern nicht biologisch abbaubar.

Eine bevorzugte Mischung für den Vliesstoff enthält mindestens 50 Gew.-% Polypropylen und / oder mindestens 20 Gew.-% Viskose. Es können auch mindestens 60 Gew.-% Polypropylen und / oder mindestens 30 Gew.-% Viskose vorliegen. Gewichtsprozente des Vliesstoffes meinen im Rahmen der Erfindung das Gewicht des reinen Vliesstoffes ohne Berücksichtigung weiterer Komponenten.

Die oben aufgeführten Fasern bzw. Faserarten haben als Vliesstoff vorliegend zum einen strukturgebende Eigenschaften und darüber hinaus die vorteilhafte Eigenschaft Flüssigkeiten wie Wundexsudat von der Wunde wegzuleiten. Dies kann durch hydrophile Eigenschaften der Fasern bewirkt werden und / oder durch physikalische Kräfte (Dochteffekt) zustande kommen.

Weiterhin kann der Vliesstoff Kunstfasern und / oder hydrophobe Fasern enthalten, die durch eine chemische oder physikalische Behandlung hydrophiliert wurden. Auf diese Weise kann der Vliesstoff von den vorteilhaften Eigenschaften von z.B. Kunstfasern - wie beispielsweise guter Prozessierbarkeit (z.B. Verschweißbarkeit), geringen Kosten und hoher Beständigkeit - profitieren, ohne dass auf die für eine erfolgreiche Wundbehandlung erforderliche hohe Absorptionskapazität verzichtet werden muss.

Wird der Vliesstoff von weiteren Schichten überlagert und bildet mit diesen zusammen eine Wundauflage, können aufgenommene Flüssigkeiten in einer oder mehreren dieser weiteren Schichten gespeichert und später mitsamt der Wundauflage entfernt werden. Bei derartigen Schichten kann es sich um Absorptionslagen handeln. Die erfindungsgemäße Wundauflage kann eine oder mehrere Absorptionslagen umfassen.

Im Allgemeinen sind Fasern natürlichen Ursprungs hydrophiler als synthetische Fasern. Dafür übertreffen synthetische Fasern die Naturfasern hinsichtlich ihrer Bindungsaffinität gegenüber unpolaren Substanzen wie Ölen, Fetten und Wachsen. Durch ihren unpolaren Charakter zeigen sie keine oder nur geringe Interaktion gegenüber den kationischen und anionischen Verbindungen der antimikrobiellen Inhaltsstoffe. Naturfasern bieten jedoch den Vorteil einer besseren Ökobilanz. Aufgrund ihrer stärkeren Absorption polarer Stoffe kann es jedoch erforderlich sein, eine größere Menge der Beschichtung aufzutragen, um eine ausreichende Beschichtungsmenge an der proximalen Seite des Vliesstoffes bereitzustellen.

In diesem Sinne kann es sich bei dem erfindungsgemäßen Vliesstoff um einen luftgelegten Vliesstoff, einen thermisch verfestigten Vliesstoff, einen mechanisch verfestigten Vliesstoff, einen Stapelfaservliesstoff, einen Schmelzblasvliesstoff, einen Spinnvliesstoff, einen Nassvliesstoff, einen Wirrlagevliesstoff (isotroper Vliesstoff) oder einen anisotropen Vliesstoff (orientierter Vliesstoff) handeln.

Die Erfindung beinhaltet eine Variante des Vliesstoffes, bei der der Vliesstoff mindestens zwei miteinander verbundene Vliesschichten unterschiedlicher Zusammensetzung umfasst. Die wundzugewandte (proximale) Vliesschicht enthält Kunstfasern und die antimikrobielle Beschichtung, während die wundabgewandte (distale) Vliesschicht Viskose enthält. Durch diese distale Viskoseschicht wird die laterale (also in Seitenansicht horizontale) Verteilung absorbierter Wundflüssigkeit verstärkt und die Absorptionsgeschwindigkeit erhöht.

Der erfindungsgemäße Vliesstoff umfasst mindestens eine Vliesschicht. Darüber hinaus kann der Vliesstoff auch zwei, höchstens zwei, drei oder höchstens drei Schichten - insbesondere Vliesschichten - umfassen, welche miteinander verbunden sind. Dies bedeutet, dass die Schichten ganz oder teilweise miteinander verbunden sind. Ganz verbunden meint dabei vollflächig. Teilweise verbunden kann beispielsweise bedeuten, dass die Schichten zumindest durchgehend im Randbereich verbunden sind, aber es im vom Randbereich umsäumten Zentrum einen unverbundenen Bereich gibt. Wenn der erfindungsgemäße Vliesstoff mehr als eine Schicht umfasst, sind diese Lagen bevorzugt vollflächig miteinander verbunden.

Wenn der Vliesstoff zwei Schichten umfasst, kann eine innere Schicht distal liegen und in Benutzung von der Wunde wegzeigen und eine äußere Schicht kann proximal liegen und in Benutzung zur Wunde ausgerichtet sein. Die äußere Schicht wird dabei in Benutzung von der inneren Schicht überlagert. Die äußere Schicht trägt bei dieser Anordnung die Beschichtung. Mindestens eine der beiden Schichten - nämlich die äußere Schicht - ist bei dieser Anordnung eine Vliesschicht. Bevorzugt sind beide Schichten Vliesschichten. In diesem Fall umfasst der erfindungsgemäße beschichtete Vliesstoff zwei Vliesschichten.

Sofern der erfindungsgemäße Vliesstoff zwei Vliesschichten umfasst, können sowohl die innere als auch die äußere Schicht jeweils mindestens 30 Gew.-%, bevorzugt mindestens 40 Gew.-%, besonders bevorzugt mindestens 50 Gew.-% und ganz besonders bevorzugt mindestens 60 Gew.-% Kunstfasern enthalten. Die prozentuellen Angaben lassen das Gewicht der antimikrobiellen Beschichtung unberücksichtigt.

Bevorzugt unterscheidet sich die innere Schicht von der äußeren Schicht hinsichtlich ihrer Zusammensetzung. Die unterschiedliche Zusammensetzung kann beispielsweise auf ganz oder teilweise unterschiedlichen Faserarten beruhen oder auf unterschiedlichen Anteilen derselben Faserarten. Dies wird im Folgenden näher ausgeführt:
Möglich ist zum Beispiel, dass sowohl die innere als auch die äußere Schicht Kunstfasern enthalten, wobei es sich bevorzugt um thermoplastische Fasern handelt. Die äußere Schicht kann sogar ausschließlich aus thermoplastischen Fasern bestehen. Besonders bevorzugt handelt es sich bei diesen thermoplastischen Fasern um Fasern aus Polypropylen und / oder Polyamid. Weiterhin kann die äußere Schicht ein Flächengewicht von beispielsweise 10 bis 30 g/m$^2$ oder 12 bis 15 g/m$^2$ haben, wobei auch hier die antimikrobielle Beschichtung unberücksichtigt bleibt.

Das Vlies der inneren und / oder der äußeren Schicht kann thermisch verfestigt sein und somit thermisch verfestigte Fasern enthalten. Auf diese Weise wird die Bindungsfestigkeit innerhalb der Schicht erhöht und gleichzeitig können leichtere Vliesstoffe generiert werden, was zu einer Materialeinsparung führt. Die Wasserdurchlässigkeit bleibt dabei erhalten. Zur Herstellung eines geeigneten thermisch verfestigten Vlieses können die zu einem lockeren Vlies gelegten Fasern bis zum Schmelzpunkt erwärmt werden, wodurch sie sich verbinden. Zum Einsatz kommen kann die Kalanderverfestigung (auch Thermobonding genannt) oder die Heißluftverfestigung (auch Thermofusion genannt), wobei im Falle von PP-Fasern sich besonders die Kalanderverfestigung bewährt hat. Thermische Verfestigung ermöglicht die Umwandlung eines losen Faservlieses in ein festes, beständiges Vlies. Das Risiko, das Fasern sich lösen und in die Wunde gelangen, ist auf diese Weise stark reduziert.

Die innere Vliesschicht kann ebenfalls Kunstfasern oder synthetische Fasern enthalten, bei denen es sich bevorzugt um thermoplastische Fasern handelt. Besonders bevorzugt enthält die innere Schicht zusätzlich zu den thermoplastischen Fasern auch andere Faserarten. Bei diesen anderen Faserarten handelt es sich bevorzugt um hydrophile Fasern, besonders bevorzugt um Zellulose-basierte Fasern und ganz besonders bevorzugt um Baumwoll- oder Viskosefasern. Auf diese Weise wird ein hygroskopischer Gradient geschaffen, der aufgenommene Flüssigkeit von der äußeren an die innere Schicht weiterreicht. Zudem fungiert die innere Schicht enthaltend hydrophile Fasern als Verteiler Schicht, was sowohl die Absorptionsgeschwindigkeit steigert, als auch die Weitergabe der Wundflüssigkeit an optionales darüberliegendes Absorptionsmaterial optimiert. Im Rahmen der vorliegenden Erfindung gelten Viskosefasern nicht als Kunstfaser oder synthetische Faser.

Eine beispielhafte Zusammensetzung der inneren Schicht ist im Folgenden wiedergegeben: 35 bis 45 Gew.-% Kunstfasern (z.B. Polypropylen und / oder Polyamid) und 55 bis 65 Gew.-% Fasern auf Zellulosebasis. Das Flächengewicht der inneren Schicht kann beispielsweise 30 bis 60 g/m$^2$ oder 40 bis 50 g/m$^2$ betragen.

Eine beispielhafte Zusammensetzung für einen erfindungsgemäßen Vliesstoff, welcher eine innere und eine äußere Schicht umfasst, kann folgendermaßen beschaffen sein: 60 bis 70 Gew.-% Kunstfasern und 30 bis 40 Gew.-% zellulosebasierte Fasern (z.B. Baumwolle oder Viskose), wobei diese Gewichtsprozente die antimikrobielle Beschichtung unberücksichtigt lassen und die innere und die äußere Schicht zusammen betrachtet werden.

Wenn sowohl die innere als auch die äußere Schicht thermoplastische Fasern enthalten, können diese durch den Einsatz von Hitze ganz oder teilweise miteinander verbunden werden, so dass sie gemeinsam den Vliesstoff ausbilden. Daneben ist auch der Einsatz von Adhäsiven wie Schmelzklebern möglich. Wenn beide Schichten als Vlies vorliegen, bietet dies den Vorteil, dass diese auf einfache Weise durch vliesspezifische Techniken wie zum Beispiel Vernadeln miteinander verbunden werden können.

Im Rahmen der Erfindung kann der Vliesstoff oder dessen Schichten bei Bedarf frei sein von bestimmten Substanzen. Beispielsweise kann der Vliesstoff frei sein von Gelatine und / oder Kollagen oder generell frei sein von Substanzen tierischen Ursprungs (z.B. Chitosan). Die Inhaltsstoffe der antimikrobiellen Beschichtung gelten in diesem Sinne nicht als Substanzen tierischen Ursprungs. Vliese, die frei sind von derartigen Stoffen zeigen im Allgemeinen eine längere Lagerungsbeständigkeit und ein geringeres Allergiepotential.

Der Vliesstoff oder dessen Schichten können beispielsweise ein Flächengewicht von 20 bis 100 g/m$^2$, bevorzugt 50 bis 100 g/m$^2$ und besonders bevorzugt 80 bis 90 g/m$^2$ haben. Derartige Vliese sind für gewöhnlich so dünn und / oder derart von Öffnungen durchbrochen, dass der Übergang von Flüssigkeit aus der Wunde durch den Vliesstoff hindurch optimiert ist. Durch den Vliesaufbau sind im Vliesstoff Öffnungen enthalten, wobei es sich hierbei um Abstände zwischen einzelnen Fasern bzw. Faserfilamenten handelt. Bei den genannten Öffnungen kann es sich um mikroskopische Öffnungen handeln, die mit dem Auge nicht erkennbar sind. Die Öffnungen erlauben den Durchtritt von Flüssigkeit. Die Öffnungen können dabei in ihrer Größe variieren und müssen nicht einheitlich sein.

Weiterhin kann die lineare Dichte des Vliesstoffes oder dessen Schichten 60 bis 100 dtex, bevorzugt 70 bis 90 dtex gemessen mittels DIN EN ISO 2060 betragen. Die Zähigkeit des Vliesstoffes kann 30 bis 50 cN/tex, bevorzugt 35 bis 40 cN/tex, gemessen mittels DIN EN ISO 2062 betragen.

Der Vliesstoff oder dessen Schichten können eine Dehnbarkeit nach DIN EN ISO 1798:2008-04 von mindestens 25%, bevorzugt von mindestens 35% haben. Dehnbarkeit ist dahingehend zu verstehen, dass es bei einer entsprechenden Verlängerung des Materials unter Zugkraft nicht zu einem Belastungsbruch oder zu einem Reißen von Fasern kommt. Bevorzugt ist die Dehnbarkeit eine reversible Verformung (Elastizität), so dass das Material nach Aufhebung der Zugkraft im Wesentlichen seine ursprüngliche Länge einnimmt. Eine reversible Dehnbarkeit liegt auch dann vor, wenn das Material nach Aufhebung der Zugkraft eine Länge einnimmt, die maximal 105% der Ausgangslänge entspricht. Die Länge hat dabei dieselbe räumliche Ausrichtung wie die Zugkraft, ist also in Richtung der Zugkraft zu messen. Bevorzugt gelten die angegebenen Dehnungswerte in Faserrichtung.

Der Vliesstoff sowie eine oder alle von dessen Schichten können optional mit elementarem, also nicht ionischem Silber behandelt sein. Dieses zusätzliche Silber, welches in den Vliesstoff eingebettet ist, dient zur Steigerung der antimikrobiellen Wirkung. Da es nicht in der Beschichtung vorhanden ist, sondern von dieser überdeckt wird, wird so eine besonders langfristige antimikrobielle Wirkung (Depoteffekt) erzeugt. Die Fähigkeit von Krankheitserregern sich im Vliesstoff anzusiedeln und zu vermehren ist weiter herabgesetzt oder sogar ganz aufgehoben. Letzteres ist besonders von Vorteil bei infizierten Wunden, die erregerhaltiges Exsudat abgeben, welches von dem Vliesstoff aufgenommen oder durch ihn hindurchgeleitet wird.

Bei dem im Rahmen der Erfindung eingesetzten Polypeptid oder Polypeptiden kann es sich um Polyarginin, Polylysin und / oder Polyornithin handeln. Somit kann jedes Polypeptidmolekül nur Aminosäuren eines einzigen Typs enthalten. Bei Polylysin kann es sich um α-Poly-L-Lysin und / oder ε-Poly-L-Lysin handeln. Das Polylysin kann beispielsweise eine Molekülmasse von 3,5 bis 4 kDa haben. Weiterhin kann das Polylysin pro Molekül 11 bis 40 Untereinheiten, bevorzugt 15 bis 35 Untereinheiten und besonders bevorzugt 20 bis 30 Untereinheiten Lysin umfassen. Ebenso kann das Polyarginin pro Molekül 11 bis 40 Untereinheiten, bevorzugt 15 bis 35 Untereinheiten und besonders bevorzugt 20 bis 30 Untereinheiten Arginin umfassen.

Alle diese genannten Polypeptide tragen aufgrund ihrer chemischen Eigenschaften eine positive Nettoladung und wirken antimikrobiell. Polyarginin hat darüber hinaus zusätzlich den Vorteil die Wundheilung zu fördern, indem der Anteil sogenannter M2 Makrophagen innerhalb der Makrophagen-Population erhöht wird. Während Makrophagen vom Typ M1 Entzündungsreaktionen initiieren und die Produktion von cytotoxischen Radikalen einleiten, wirken M2 Makrophagen entzündungshemmend, proliferativ und fördern den Gewebeverschluss.

Bei Mischungen des Polypeptids kann es sich insbesondere um folgende Mischungen handeln: a) Polyarginin in Kombination mit Polylysin, b) Polyarginin in Kombination mit Polyornithin, c) Polylysin in Kombination mit Polyornithin sowie d) eine Mischung aus allen drei dieser Verbindungen. Das Polypeptid bzw. die Polypeptide sind Teil der erfindungsgemäßen Beschichtung. Dabei kann die Anzahl der Moleküle für die unterschiedlichen Arten von Polypeptiden variabel sein. Auch können in der Beschichtung Polypeptide mit unterschiedlichen Kettenlängen vorhanden sein. Hierzu wird an anderer Stelle im Detail eingegangen.

Weiterhin können die Polypeptide Mischungen der drei zuvor genannten Aminosäuren in einem Molekül enthalten. Dabei können die Aminosäuren Polyarginin und Polylysin oder aber Polyarginin und Polyornithin oder Polylysin und Polyornithin in einem Polypeptid enthalten sein oder das Polypeptid enthält alle drei Aminosäuren.

Bevorzugt enthält die erfindungsgemäße Beschichtung Polyarginin. Der Einsatz derartiger erfindungsgemäßer polyargininhaltiger Beschichtungen ist selbst bei nicht infizierten Wunden vorteilhaft, da die Heilung beschleunigt wird.

Darüber hinaus bietet die Kombination aus Polyarginin und Polylysin in der Beschichtung eine besonders ausgeprägte antimikrobielle Wirkung, die vermutlich auf einen synergistischen Effekt zurückzuführen ist und die antimikrobielle Wirkung der jeweiligen Einzelsubstanz gegenüber diversen Erregern zu übertreffen vermag.

Alternativ kann das Polypeptid ein solches Polypeptid sein, das im Wesentlichen in einer einzigen Kettenlänge oder ausschließlich einer einzigen Kettenlänge vorhanden ist. Eine im Wesentlichen einzige Kettenlänge liegt dann vor, wenn mindestens 90 %, besser mindestens 95 %, am besten mindestens 98 % und am allerbesten mindestens 99 % aller in der Beschichtung enthaltenen Polypeptidmoleküle dieselbe Kettenlänge haben. Eine Beschichtung enthaltend Polypeptidmoleküle derselben oder im Wesentlichen derselben Kettenlänge bietet den Vorteil, dass die antibakterielle Wirkung und die Stabilität der Beschichtung sich sehr gut vorhersagen lassen.

Bevorzugt liegt die Anzahl der Aminosäuren in dem Polypeptid oder in den Polypeptiden bei mindestens 10. Weiterhin liegt die Anzahl der Aminosäuren in einem Polypeptid oder in den Polypeptiden bevorzugt bei höchstens 2000. Somit haben Polypeptid oder Polypeptide im Rahmen der Erfindung eine bevorzugte Kettenlänge von 10 bis 2000 Aminosäuren, besonders bevorzugt 20 bis 1000 Aminosäuren, ganz besonders bevorzugt 25 bis 100 Aminosäuren und am besten 30 bis 50 Aminosäuren. Wie aus den Ausführungsbeispielen hervorgeht, können auch sämtliche Polypeptide in der Beschichtung jeweils 30 Aminosäuren enthalten oder aus 30 Aminosäuren bestehen. Der daraus resultierende Vorteil ist eine vereinfachte Bereitstellung bei gleichzeitig stark ausgeprägter antimikrobieller Wirkung.

Gemäß einem Aspekt der Erfindung umfassen das Polypeptid oder die Polypeptide mindestens zehn und / oder höchstens hundert Aminosäuren. Diese Anzahl bezieht sich auf die Menge an Aminosäuren pro Molekül eines Polypeptids. Bevorzugt können das Polypeptid oder die Polypeptide höchsten neunzig, insbesondere höchstens siebzig, besonders bevorzugt höchstens fünfzig und am besten höchstens vierzig Aminosäuren umfassen. Gleichzeitig oder auch unabhängig von der genannten Obergrenze können das Polypeptid oder die Polypeptide in der Beschichtung mindestens zehn Aminosäuren umfassen. Bevorzugt können das Polypeptid oder die Polypeptide mindesten 20, besonders bevorzugt mindestens 25 und am besten mindestens 28 Aminosäuren umfassen. Die genannten Zahlen beziehen sich auf die Menge an Aminosäuren pro Molekül eines Polypeptids. Typischerweise sind diese Aminosäuren über Peptidbindungen miteinander verbunden. Die genannten Werte können sich auf einen Teil der Polypeptide in der Beschichtung (z.B. mindestens 90 Gew.-%) oder auf sämtliche Polypeptide in der Beschichtung beziehen.

Somit kann im Rahmen der Erfindung der Vliesstoff eine Beschichtung aufweisen, enthaltend ein Polypeptid (Polyarginin, Polylysin, Polyornithin oder eine der oben beschriebenen Mischungen daraus), das mindestens zehn Aminosäuren und/oder höchstens hundert Aminosäuren umfasst. Alternativ kann das Polypeptid mindestens zwanzig und / oder höchsten achtzig Aminosäuren umfassen. Bevorzugt umfasst das Polypeptid mindestens fünfundzwanzig und / oder fünfzig Aminosäuren. Diese Anzahl an Aminosäuren kann für einen Teil der Polypeptidmoleküle gelten oder kann alternativ auch für sämtliche Polypeptidmoleküle in der Beschichtung gelten.

Hyaluronsäure, auch bekannt als Hyaluronan, ist ein zu den Glycosaminoglycanen zählendes Heteropolysaccharid. Grundbaustein einer Hyaluronsäure ist ein aus d-Glucuronsäure und N-Acetyl-d-glucosamin alternierend in (1→3)-(1→4)-β-glycosidischer Bindung aufgebautes Aminodisaccharid. Hyaluronsäuren sind Teil der erfindungsgemäßen Beschichtung und tragen aufgrund ihrer chemischen Eigenschaften eine negative Nettoladung. Als negativ geladenes Polymer gehören Hyaluronsäuren zu den Polyanionen. Hyaluronsäuren sind wasserbindend und haben gewebsregenerierende und wundheilende Eigenschaften. Eine Möglichkeit eine Hyaluronsäure zu erhalten, ist diese zu synthetisieren, indem Proteine einer bakteriellen Fermentierung unterzogen werden. Durch eine anschließende Filtrierung wird eine reine Hyaluronsäure gewonnen. Die im Rahmen der vorliegenden Erfindung zu nutzenden Hyaluronsäuren sind hydrophil und somit löslich in Wasser und den meisten anderen polaren Substanzen.

Hyaluronsäuren können im Rahmen dieser Erfindung in Molmassen von ca. 50 bis ca. $10^4$ kg / mol verwendet werden, bevorzugt werden Hyaluronsäuren mit einer molaren Masse von 140 bis 150 kg / mol verwendet. Im Rahmen dieser Erfindung ist es auch möglich, eine Mischung von Hyaluronsäuremolekülen unterschiedlicher Molmassen zu verwenden, wobei in diesem Fall die Molmasse als durchschnittliche Molmasse (Durchschnittsmolmasse) aller Hyaluronsäuremoleküle in der Mischung oder Beschichtung angegeben werden kann. Beispielsweise kann die durchschnittliche Molmasse 143 bis 146 kg / mol betragen. Alternativ haben alle oder im Wesentlichen alle Hyaluronsäuremoleküle in der Beschichtung dieselbe Molmasse. Eine im Wesentlichen selbe Molmasse liegt vor, wenn mindestens 90 %, besser mindestens 95 %, noch besser mindestens 98 % und am besten mindestens 99 % der Hyaluronsäuremoleküle in der Beschichtung dieselbe Molmasse haben. Generell gilt, dass geeignete Messmethoden zur Molmassenbestimmung wie z.B. die Massenspektrometrie aus dem Stand der Technik bekannt sind.

Hyaluronsäuren können quervernetzt oder unvernetzt vorliegen, wobei unvernetzte Hyaluronsäuren bevorzugt sind. Gleichzeitig kann auch die antimikrobielle Beschichtung quervernetzt oder unvernetzt vorliegen, wobei unvernetzte Beschichtungen bevorzugt sind. Mögliche Verfahren zur Quervernetzung von Hyaluronsäuren sind der Einsatz von 1,4-Butandioldiglycidylether (BDDE), enzymatische Quervernetzung (z.B. mittels Transglutaminasen) oder physikalische Quervernetzung (z.B. mittels Gefrieren, Erhitzen, Ultraschall, Mikrowellen). Ein mögliches Verfahren zur Quervernetzung von Hyaluronsäuren wird in der WO2010131175A1 beschrieben.

Die erfindungsgemäße Beschichtung enthält mindestens eine hierin beschriebene Hyaluronsäure und das hierin beschriebene Polypeptid bzw. die Polypeptide. Weitere Verbindungen oder strukturelle Komponenten können ebenfalls Teil der Beschichtung sein oder mit dieser kombiniert werden. Diese weiteren Verbindungen oder strukturellen Komponenten können flüssig oder fest sein. Darüber hinaus können sie positiv geladen, negativ geladen oder ladungsneutral sein.

In diesem Sinne kann die Beschichtung eine polare Flüssigkeit enthalten. Bei der polaren Flüssigkeit kann es sich um Wasser handeln. Das Wasser kann als destilliertes oder deionisiertes Wasser vorliegen. Bevorzugt liegt die polare Flüssigkeit (z.B. Wasser) als wässrige Pufferlösung vor. Beispiele für wässrige Puffer sind Citratpuffer, Ringerlösung, TRIS-Puffer, TE-Puffer, TBS-Puffer und TBS-T-Puffer. Bevorzugt handelt es sich bei dem Puffer um Tris-NaCl-Puffer. Die Konzentration des Puffers im Lösungsmittel (z. B. Wasser) kann beispielsweise 5 mmol bis 300 mmol betragen, bevorzugt beträgt die Konzentration 10 mmol bis 200 mmol. Im Falle von Tris-NaCl-Puffer kann die Konzentration von Tris beispielsweise 5 bis 300 mmol und die Konzentration von NaCl 10 mmol bis 300 mmol betragen. Alternativ kann die Konzentration des Puffers so gewählt werden, dass die gepufferte Lösung einen pH von 6,8 bis 7,8, bevorzugt 7,2 bis 7,6 hat. Diese pH-Werte beziehen sich zunächst auf die Lösung vor ihrer Vermengung mit anderen Bestandteilen der Beschichtung. Allerdings sind diese Werte auch auf die fertige Beschichtung im finalen Produkt - also dem erfindungsgemäßen Vliesstoff - anwendbar, und zwar sowohl vor als auch nach einer optionalen Trocknung.

Die Masse der bereits erwähnten Puffersubstanzen in Form einer Base oder einer Säure sowie einem geeigneten Salz dieser Base oder Säure kann eine Erhöhung des Flächengewichts in der Beschichtung um beispielsweise 50 ng bis 1.000 ng / cm$^2$ Vliesstoff bewirken. Bevorzugt haben die Puffersubstanzen in der Beschichtung ein Flächengewicht von 100 ng bis 500 ng / cm$^2$ Vliesstoff, besonders bevorzugt ein Flächengewicht von 120 ng bis 300 ng / cm$^2$ Vliesstoff. Bevorzugt enthält die Puffersubstanz eine Base, besonders bevorzugt handelt es sich um Tris und ganz besonders bevorzugt wird die Base Tris kombiniert mit dem Salz NaCl.

Die Beschichtung kann einen Konservierungsstoff oder einen Stabilisator in einer Menge von 0,1 bis 2 Gew.-% enthalten. Beispiele für geeignete Konservierungsstoffe sind beispielsweise Benzoesäure, Sorbinsäure oder Parabene. Beispiele für geeignete Stabilisatoren sind L-Ascorbylpalmitat und Tocopherol. Da der Vliesstoff mitsamt Beschichtung in der Regel vor der Verwendung sterilisiert wird, kann in den meisten Fällen auf die Verwendung von Konservierungsstoffen verzichtet werden, um den Produktionsaufwand gering zu halten. Vorzugsweise kann auch vorgesehen sein, dass die Beschichtung generell keine Konservierungsstoffe und / oder Stabilisatoren enthält, um die Wahrscheinlichkeit für das Auftreten von Allergien und Unverträglichkeitsreaktionen zu reduzieren. In diesem Sinne kann die erfindungsgemäße Beschichtung und der damit ausgestattete Vliesstoff antiallergen sein.

Der Gehalt an polaren Flüssigkeiten, insbesondere an Wasser, in der Beschichtung kann 0,1 bis 50 Gew.-% betragen. In manchen Fällen (z.B., wenn die Beschichtung als Gel vorliegen soll) können auch mehr als 50 Gew.-% an polaren Flüssigkeiten gewünscht und sinnvoll sein. Bevorzugt enthält die Beschichtung 1 bis 45 Gew.-% polare Flüssigkeiten, besser 3 bis 40 Gew.-% polare Flüssigkeiten und am besten enthält die Beschichtung 5 bis 35 Gew.-% polare Flüssigkeiten. Diese Konzentrationen können sich auf den finalen Gehalt im fertigen Produkt nach aktiver oder passiver Trocknung beziehen. Es können zwei oder mehr verschiedene polare Flüssigkeiten in der Beschichtung vorhanden sein. Beispiele für mögliche Kombinationen aus polaren Flüssigkeiten sind Wasser und Ethanol oder Wasser und Glycerin. Darüber hinaus kann es sich bei der polaren Flüssigkeit um einen flüssige Pufferlösung wie beispielsweise Tris-NaCl-Puffer handeln. Die polare Flüssigkeit bzw. die Pufferlösung können hierbei einen pH von 6 bis 9, bevorzugt 7 bis 8 und besonders bevorzugt 7,2 bis 7,6 haben.

Die erfindungsgemäße Beschichtung ist eine stabile Beschichtung und bietet eine hervorragende Langzeithaltbarkeit. Diese Langzeitstabilität ist bei medizinischen Artikeln eine besonders gewünschte Eigenschaft, da derartige Produkte von medizinischen Einrichtungen im Rahmen rabattierter Großbestellungen erworben werden und bis zu ihrem Einsatz (dessen Zeitpunkt im Regelfall nicht absehbar ist) gelagert werden müssen. Während der Lagerung können die Produkte durchaus saisonal bedingten Temperaturschwankungen unterliegen. Die erfindungsgemäße Beschichtung gewährleistet Beständigkeit sowohl bei langer Lagerungsdauer als auch gegenüber Temperaturschwankungen. Letzteres ist auch einem möglichen Sterilisationsprozess zuträglich.

Die Beschichtung und somit der beschichtete Vliesstoff hat antimikrobielle, insbesondere antibakterielle Eigenschaften, wobei die antibakteriellen Eigenschaften eine Wirkung gegen die humanpathogenen Keime *S. aureus* sowie *P. aeruginosa* umfassen. Die antimikrobielle Wirkung tritt bereits beim Initialkontakt mit den Erregern ein und kann sich im Laufe der Zeit verstärken, wobei eine Kontaktzeit (z.B. Anwendungsdauer auf einer Wunde) von 0 bis 24 Stunden ein bevorzugter Wirkungszeitraum ist.

Gemäß einem weiteren Aspekt der Erfindung liegt die Hyaluronsäure in der Beschichtung als Polymer vor, wobei es sich um eine Polymermischung mit unterschiedlicher Kettenlänge handeln kann. Dabei kann zumindest ein Teil dieser Polymere eine Molekülmasse von mindestens 10 kDa haben. Bevorzugt hat zumindest ein Teil dieser Polymere der Hyaluronsäure eine Molekülmasse von mindestens 15 kDa, besonders bevorzugt von mindestens 20 kDa, ganz besonders bevorzugt von mindestens 25 kDa und am besten von mindestens 30 kDa. Dieser Teil kann z.B. mindestens 90 Gew.-% der Hyaluronsäure in der Beschichtung ausmachen, bevorzugt mindestens 95 Gew.-% der Hyaluronsäure, besonders bevorzugt mindestens 99 Gew.-% der Hyaluronsäure oder die angegebenen Werte können sich alternativ auf die gesamte Hyaluronsäure in der Beschichtung beziehen.

Im Rahmen der Erfindung kann die Hyaluronsäure als Polymermischung mit unterschiedlicher Kettenlänge vorliegen, wobei zumindest ein Teil der Hyaluronsäurepolymere in der Polymermischung eine Molekülmasse von mindestens 10 kDa und / oder höchstens 300 kDa hat. Bevorzugt hat zumindest ein Teil dieser Polymere der Hyaluronsäure eine Molekülmasse von höchstens 250 kDa, besonders bevorzugt höchstens 200 kDa, ganz besonders bevorzugt höchstens 150 kDa und am besten höchstens 100 kDa. Dieser Teil kann z.B. 90 Gew.-% der Hyaluronsäure in der Beschichtung ausmachen oder die angegebenen Werte können sich alternativ auf die gesamte Hyaluronsäure in der Beschichtung beziehen.

Weiterhin kann die Beschichtung des erfindungsgemäßen Vliesstoffes einen Aufbau aus zwei oder mehr Lagen aufweisen. Der Begriff Lage bezieht sich auf eine Schicht innerhalb der Beschichtung. Eine Lage kann in einem Beschichtungsschritt aufgebracht werden. Die erste Lage wird auf den Vliesstoff aufgebracht, wobei hierbei üblicherweise die proximale Oberfläche des Vliesstoffs behandelt wird. Jede weitere Lage - angefangen mit der zweiten Lage - wird auf die zuletzt aufgebrachte Lage aufgebracht. Dabei kann eine Lage entweder die Hyaluronsäure enthalten und hat somit eine negative Nettoladung oder kann das Polypeptid bzw. die Polypeptide enthalten und damit eine positive Nettoladung haben. Hierbei kann vorgesehen sein, dass eine Lage, die die Hyaluronsäure enthält, frei ist von dem Polypeptid bzw. den Polypeptiden und eine Lage, die das Polypeptid bzw. die Polypeptide enthält, frei ist von der Hyaluronsäure. Im Unterschied hierzu kann die Beschichtung auch durchmischte Lagen enthalten, auf welche an anderer Stelle näher eingegangen wird.

Die Beschichtung kann mindestens zwei übereinanderliegende und miteinander verbundenen Lagen enthalten, wobei mindestens eine Lage vorhanden ist, welche das Polypeptid oder die Polypeptide enthält und eine positive Nettoladung hat sowie mindestens eine Lage, welche eine Hyaluronsäure enthält und eine negative Nettoladung hat, und wobei sich bei den übereinanderliegenden Lagen jeweils eine das Polypeptid oder die Polypeptide enthaltende Lage mit einer eine Hyaluronsäure enthaltenden Lage abwechselt, so dass eine Abfolge von alternierenden Lagen gebildet wird.

Die Anzahl der alternierenden Lagen kann dabei eine gerade oder ungerade Zahl sein. Eine gerade Anzahl an alternierenden Lagen ist dabei bevorzugt, weil dabei für jede negativ geladene Lage eine positiv geladene Lage zur Verfügung steht und sich die gegensätzlichen Ladungen anziehen, was zu einer besonders stabilen Beschichtung führt.

Vorzugsweise ist die Anzahl der alternierenden Lagen 20 bis 100, bevorzugt 30 bis 90, besonders bevorzugt 40 bis 80 und ganz besonders bevorzugt 50 bis 70. Dabei enthält jeweils die Hälfte der Lagen eine Hyaluronsäure und die andere Hälfte der Lagen das Polypeptid oder die Polypeptide. Bei einer ungeraden Anzahl an alternierenden Lagen überwiegt bevorzugt die Anzahl derjenigen Lagen, die das Polypeptid enthalten.

Darüber hinaus kann die Anzahl der übereinanderliegenden und miteinander verbundenen Lagen in der Beschichtung beispielsweise 10 bis 100 betragen. Bevorzugt beträgt die Anzahl der übereinanderliegenden und miteinander verbundenen Lagen 15 bis 90, besonders bevorzugt 20 bis 80, ganz besonders bevorzugt 25 bis 70 und am besten 30 bis 60.

Eine Möglichkeit die Lagen der Beschichtung aufzutragen, besteht darin, diese mittels Tauchverfahren aufzubringen. Das Tauchverfahren ist darüber hinaus das geeignete Verfahren, um solche Lagen innerhalb der Beschichtung zu erzeugen, die jeweils entweder eine Hyaluronsäure ohne ein Polypeptid oder ein Polypeptid ohne eine Hyaluronsäure enthalten. So ist es beim Tauchverfahren möglich zwei Lösungen in zwei separaten Kompartimenten bereitzustellen, wobei ein Kompartiment die Lösung mit der Hyaluronsäure und das andere Kompartiment die Lösung mit dem Polypeptid bzw. den Polypeptiden enthält. Auf den Vliesstoff können auf diese Weise durch abwechselndes Eintauchen in den beiden Kompartimenten Lagen aufgetragen werden, bis die Beschichtung vollständig ist.

Im Rahmen der Erfindung können die Hyaluronsäure und das Polypeptid bzw. die Polypeptide innerhalb der Beschichtung oder innerhalb einer Lage der Beschichtung jedoch auch durchmischt vorliegen. Durchmischt ist in diesem Sinne als Gemisch im chemischen Sinne zu verstehen. Das Gemisch ist hierbei bevorzugt eine Lösung, und zwar eine Lösung mit nur einer Phase. Bevorzugt sind die Hyaluronsäure und das Polypeptid bzw. die Polypeptide innerhalb der Beschichtung homogen verteilt. Weiterhin bevorzugt ist das Polypeptid oder sind die Polypeptide in eine Hyaluronsäurematrix eingebettet. Eine solche Matrix kann als Hyaluronsäuregel vorliegen und Hyaluronsäure sowie Wasser enthalten. Die Hyaluronsäurematrix bildet sich aus, wenn die Beschichtung im trockenen Zustand vorliegt oder durch Trocknung in diesen übergeht, wobei eine Restfeuchtigkeit durch chemische Wechselwirkungen erhalten bleibt.

Eine durchmischte Beschichtung im Sinne des eben Genannten lässt sich durch Aufsprühen einer Lösung enthaltend Hyaluronsäure und Aufsprühen einer Lösung enthaltend das Polypeptid bzw. die Polypeptide auf den Vliesstoff erhalten. Die beiden Lösungen können nacheinander oder gleichzeitig gesprüht werden. Mögliche Verfahren sind in den Ausführungsbeispielen näher erläutert. Sobald eine Lage erhalten wurde und diese getrocknet oder angetrocknet ist, können weitere Lagen aufgesprüht oder mittels Tauchverfahren hinzugefügt werden.

Eine durchmischte Beschichtung kann eine oder mehrere Lagen enthalten, von denen mindestens eine Lage sowohl eine Hyaluronsäure als auch das Polypeptid bzw. die Polypeptide enthält. In diesem Sinne ist eine solche durchmischte Beschichtung gänzlich oder möglicherweise nur teilweise von der Mischung durchdrungen.

Somit können auch eine oder mehrere Lagen einer Beschichtung durchmischt sein, wobei eine solche durchmischte Lage sowohl die Hyaluronsäure als auch ein oder mehrere der besagten Polypeptide enthält.

Der Vliesstoff kann die antimikrobielle Beschichtung in folgenden Varianten enthalten, wobei diese Aufzählung exemplarisch und nicht abschließend zu verstehen ist:

a) Beschichtung umfassend mindestens zwei Lagen, von denen mindestens eine Lage eine Hyaluronsäure, aber kein Polypeptid enthält und von denen mindestens eine Lage ein Polypeptid oder Polypeptide aber keine Hyaluronsäure enthält. Falls mehr als zwei Lagen vorhanden sind, sind diese alternierend angeordnet.
b) Durchmischte Beschichtung umfassend mindestens eine Lage, die sowohl eine Hyaluronsäure als auch das Polypeptid bzw. die Polypeptide enthält.
c) Eine Kombination aus a) und b).

In diesem Sinne umfasst die Erfindung einen Vliesstoff, der beidseitig beschichtet ist und bei der beide Seiten unabhängig voneinander mit einer Beschichtung gemäß a), b) oder c) ausgestattet sind.

Sofern die proximale und die distale Seite des Vliesstoffes mit der Beschichtung beschichtet sind, bietet das den Vorteil, dass der Anwender nicht entscheiden muss, welche Seite zum Auflegen auf die Wunde konfiguriert ist, was die Wahrscheinlichkeit von Fehlern reduziert und die Arbeit im klinischen Alltag erleichtert. Falls nur eine Seite des Vliesstoffes beschichtet ist, kann die distale oder proximale Seite des Vliesstoffes durch Farbgebung als Ober- oder Unterseite gekennzeichnet sein.

Gemäß einem Konzept der Erfindung haben das Polypeptid bzw. die Polypeptide eine Molekülmasse von 1 bis 41 kDa. Bevorzugt haben das Polypeptid bzw. die Polypeptide eine Molekülmasse von 2 bis 40 kDa, besonders bevorzugt 3 bis 39 kDa, ganz besonders bevorzugt 4 bis 38 kDa und am besten 5 bis 37 kDa. Möglich ist auch, dass mindestens eine, mindestens zwei oder mindestens drei Lagen der Beschichtung ein Polypeptid bzw. Polypeptide mit einer solchen Molekülmasse hat.

Die Beschichtung des Vliesstoffes kann eine Dicke von 10 nm bis 1000 nm haben. Bevorzugt hat die Beschichtung eine Dicke von 50 nm bis 900 nm, besonders bevorzugt eine Dicke von 100 nm bis 800 nm, besonders bevorzugt eine Dicke von 150 nm bis 700 nm und am besten 200 nm bis 600 nm. Die Dicke kann dabei gemessen werden von der Oberkante der Grundfläche des Vliesstoffes, und zwar ohne, dass der Vliesstoff während der Messung gestaucht oder komprimiert wird. Die Werte beziehen sich auf die Dicke der Beschichtung nach aktiver oder passiver Rücktrocknung.

Es wird empfohlen die Dicke der Beschichtung durch die Anzahl der Lagen einzustellen. Die Dicke nimmt mit zunehmender Anzahl der Lagen weiter zu. Beschichtungen, deren Dicke eine größere Ausdehnung erreicht, haben besonders ausgeprägte atraumatische Eigenschaften. Beschichtungen, die eine geringe Dicke aufweisen, ermöglichen einen schnelleren Durchfluss von Flüssigkeiten aus der Wunde in den Vliesstoff.

Der erfindungsgemäße Vliesstoff kann eine Beschichtung mit einem Flächengewicht von beispielsweise 5 ng bis 200 ng / cm$^2$ enthalten. Bevorzugt hat die Beschichtung ein Flächengewicht von 10 ng bis 150 ng / cm$^2$, besonders bevorzugt ein Flächengewicht von 15 ng bis 130 ng / cm$^2$, ganz besonders bevorzugt ein Flächengewicht von 20 ng bis 100 ng / cm$^2$ und am besten 20 ng bis 50 ng / cm$^2$. Das Flächengewicht der Beschichtung bezieht sich dabei auf die Summe der Massen aus Polypeptid bzw. Polypeptiden und Hyaluronsäure.

Das Flächengewicht des Polypeptids bzw. der Polypeptide in der Beschichtung kann beispielsweise 1 ng bis 500 ng / cm$^2$, bevorzugt 2 ng bis 300 ng / cm$^2$ und ganz besonders bevorzugt 3 ng bis 50 ng / cm$^2$ Vliesstoff betragen.

Das Flächengewicht der Hyaluronsäure in der Beschichtung kann beispielsweise 3 ng bis 1.000 mg / cm$^2$, bevorzugt 6 ng bis 400 ng / cm$^2$ und besonders bevorzugt 7 ng bis 50 ng / cm$^2$ Vliesstoff betragen.

Das Verhältnis von Polypeptid bzw. Polypeptiden zu Hyaluronsäure in der Beschichtung kann beispielsweise ein Massenverhältnis von 0,5 zu 1 bis 5 zu 1 haben. Bevorzugt liegt das Verhältnis bei 1 zu 1 bis 4 zu 1, besonders bevorzugt liegt das Verhältnis bei 1,5 zu 1 bis 3,5 zu 1 und ganz besonders bevorzugt bei 2 zu 1 bis 3,5 zu 1.

Ferner kann das Stoffmengenverhältnis der Stoffmenge von Hyaluronsäure zur Stoffmenge des Polypeptids oder der Stoffmengen der Polypeptide von 4 zu 25 bis hin zu 1 zu 500 reichen. Die Stoffmengenbestimmung kann in der Standardeinheit mol vorgenommen werden.

Der Vliesstoff kann teilweise oder vollständig mit der Beschichtung beschichtet sein. Insbesondere können mindestens 80 % der Fläche der proximalen Seite des Vliesstoffes beschichtet sein. Bevorzugt sind 90 % der Fläche der proximalen Seite des Vliesstoffes beschichtet. Besonders empfohlen wird mindestens 99 % dieser Fläche zu beschichten. In diesem Zusammenhang sei darauf hingewiesen, dass die Beschichtung ein stabiler Teil des behandelten Vliesstoffes ist, aber für Wasser bzw. wässrige Wundflüssigkeiten passierbar ist. Auf diese Weise können überschüssiges Wundexsudat oder Blut in optional vorhandenen weiteren Schichten, die sich distal zum Vliesstoff befinden, gespeichert werden. Mithilfe absorbierender Materialien kann dabei ein Sogeffekt und / oder Dochteffekt generiert werden.

Die applizierte Beschichtungsmenge kann durch Wiegen des behandelten Vliesstoffes ermittelt werden. Beschichtungen mit größerem Flächengewicht können durch wiederholte Einzelbeschichtungen erzeugt werden.

Weiterhin ist die erfindungsgemäße Beschichtung des Vliesstoffes vorzugsweise trocknungsbeständig. In diesem Sinne können die Bestandteile der Beschichtung - wie Hyaluronsäure und Polypeptid oder Polypeptide - im feuchtem Zustand als Lösung aufgetragen werden, um anschließend zu trocknen. Das Trocknen kann passiv bei Raumtemperatur erfolgen oder aktiv unter Einsatz technischer Hilfsmittel, wobei letzteres im Allgemeinen deutlich schneller, aber auch energieaufwändiger ist. Die Beschichtung behält auch im getrockneten Zustand ihre Eigenschaften - insbesondere die atraumatische und antimikrobielle Wirkung - bei. Die Trocknungsbeständigkeit der Beschichtung hat den Vorteil, dass sich trockene Wundversorgungsprodukte einfacher im industriellen Maßstab verarbeiten und verpacken lassen. Darüber hinaus lassen sich trocknungsbeständige Wundversorgungsprodukte leichter und länger lagern, da sie nicht gegen ein ungewolltes Eintrocknen geschützt werden müssen.

Die Beschichtung gilt beispielsweise als trocken, wenn der Flüssigkeitsgehalt (z.B. Wassergehalt) in der Beschichtung maximal 5 Gew.-%, bevorzugt maximal 4 Gew.-%, besonders bevorzugt maximal 3 Gew.-%, ganz besonders bevorzugt maximal 1 Gew.-% und am besten maximal 0,1 Gew.-% beträgt. Geringe Abweichungen von diesen Werten können sich in Abhängigkeit von der Konzentration gelöster Teilchen, deren Hydrophilie und Molmasse ergeben.

Überraschenderweise hat sich gezeigt, dass die Beschichtung und der mit der Beschichtung ausgestattete Vliesstoff durchleitend für Wundexsudat sind. Die Erfindung umfasst in diesem Sinne auch einen beschichteten Vliesstoff, der durchlässig oder durchleitend für polare Flüssigkeiten wie Wundexsudat oder Blut ist. Dies erlaubt die Platzierung weiterer saugfähiger Materialien oberhalb (distal) vom beschichteten Vliesstoff, welche das hindurchtretende Exsudat speichern. Hierbei ist es vorteilhaft, wenn die Faser oder die Fasermischung des Vliesstoffes zumindest im Bereich der beschichteten Oberfläche (proximale Seite) hydrophobe Eigenschaften hat. Diese können dadurch vermittelt werden, dass synthetische Fasern (z.B. aus Polypropylen) oder ein Anteil von synthetischen Fasern innerhalb einer Fasermischung verwendet wird. Ein solcher hydrophober Vliesstoff leitet Wundexsudat an distal gelegene hydrophile Materialien weiter. Ein solcher Aufbau ist insbesondere bei der erfindungsgemäßen Wundauflage möglich und kann sich bei mehrschichtigen Vliesstoffen auf die proximale Vliesschicht beziehen. Weitere Details zum Aufbau der erfindungsgemäßen Wundauflage sind an anderer Stelle erläutert.

Die proximale und optional auch die distale Seite des Vliesstoffes kann mit einer Beschichtung mit dem oben genannten Flächengewicht ausgestattet sein. Es hat sich gezeigt, dass die vorgeschlagene Menge an antimikrobieller Beschichtung zu hervorragenden Ergebnissen bei der Förderung der Wundheilung und der Bekämpfung von Krankheitserregern in der Wunde führt. Gleichzeitig ist es möglich, ein stabiles Produkt mit ausreichend langer Lagerfähigkeit zu erhalten, und zwar ohne, dass sich die Beschichtung vom Vliesstoff löst. Bevorzugt handelt es sich bei der Beschichtung um eine gleichmäßige oder im Wesentlichen gleichmäßige Verteilung der Beschichtungsmasse, bei der jeder Bereich der präparierten Seite des Vliesstoffes mit derselben oder im Wesentlichen derselben Menge an Beschichtung ausgestattet ist.

Die Erfindung betrifft darüber hinaus eine Wundauflage, die den erfindungsgemäßen Vliesstoff umfasst. Der Vliesstoff liegt dabei als eine proximale Außenschicht der Wundauflage vor, so dass zumindest ein Teil der Beschichtung während der Anwendung in Kontakt mit der Haut bzw. der Wunde kommt (proximale Ausrichtung der Beschichtung innerhalb des Vliesstoffs der Wundauflage). Ein permanenter Kontakt während der Anwendung ist dabei nicht notwendig, aber vorteilhaft.

Bei der Wundauflage kann es sich um einen absorbierenden oder superabsorbierenden Verband, einen Inselverband, einen sogenannten Sandwichverband, ein Pflaster, einen Kompressionsverband oder eine Kompresse handeln. Der beschichtete Vliesstoff kann innerhalb der Wundauflage beispielsweise als Wundpad vorliegen (z.B. bei einem Pflaster) oder in anderen Konstellationen, die weiter unten im Detail beschrieben werden.

Weiterhin kann die Wundauflage bzw. die eben genannten Ausgestaltungformen einer Wundauflage über adhäsive Mittel zur Klebebefestigung an der Haut verfügen, was jedoch nicht zwingend erforderlich ist. Der Einsatz oder Einbau adhäsiver Mittel wird weiter unten anhand einer Ausführungsform näher erläutert.

Vor der Verwendung kann die beschichtete Seite des Vliesstoffes herstellerseitig durch eine Schutzschicht wie beispielsweise einen Release-Liner abgedeckt sein. Eine solche Schutzschicht wird vom Anwender vor der Verwendung entfernt.

Bei der oben beschriebenen Wundauflage kann vorgesehen sein, dass der beschichtete Vliesstoff Teil eines Wundkissens, einer Tasche oder eines Saugkörpers ist, wobei das Wundkissen, die Tasche oder der Saugkörper ein hydrophiles, absorbierendes Material enthält. Dabei kann der Vliesstoff mindestens einen Teil der proximalen Seite des Wundkissens, der Tasche oder des Saugkörpers ausbilden. Das Wundkissen, die Tasche oder der Saugkörper kann ein hydrophiles, absorbierendes Material umfassen oder enthalten.

Bei dem hydrophilen, absorbierenden Material kann es sich um ein Material handeln, dass eine superabsorbierende Substanz umfasst. Die superabsorbierende Substanz kann ein superabsorbierendes Polymer sein und kann (insbesondere im trockenen Zustand) in Form von Partikeln und / oder Fasern vorliegen. Weiterhin kann diese superabsorbierende Substanz innerhalb des Wundkissens, der Tasche oder des Saugkörpers vermischt mit einem oder mehreren anderen hydrophilen, absorbierenden Materialien vorliegen. Bei dem oder den anderen hydrophilen, absorbierenden Materialien handelt es sich bevorzugt um Fasern oder Flocken aus Zellulose, Baumwolle und / oder Viskose.

Der Vliesstoff macht dabei zumindest einen Teil der proximalen Seite des Wundkissens oder Saugkörpers aus, wobei das Wundkissen oder der Saugkörper Bestandteil der Wundauflage ist und in der Wundauflage enthalten ist.

Weiterhin kann die genannte Wundauflage ein Backing als distale Abschlussschicht beinhalten, wobei die proximale Seite dieses Backings mit einer adhäsiven Beschichtung ausgestattet ist und wobei die Flächenausdehnung des Backings andere Bestandteile der Wundauflage überragt, so dass ein umlaufender Kleberand ausgebildet wird, der während des Gebrauchs eine Anhaftung der Wundauflage - z.B. an die Haut eines Patienten - ermöglicht. Das Backing selbst kann aus einer Folie oder einem Vliesmaterial bestehen. Ein Backing, das als Folie ausgestaltet ist, kann derart ausgestaltet sein, dass es undurchdringlich für Wasser ist, aber durchlässig gegenüber Wasserdampf. Im Falle einer Folie kann diese Polyurethan beinhalten oder daraus bestehen. Im Falle des Vliesmaterials kann dieses aus den gleichen oder anderen Fasern oder Fasergemischen bestehen wie der erfindungsgemäße Vliesstoff. Da das Backing nicht dafür vorgesehen ist, mit der Wunde in Kontakt zu kommen, benötigt es keine antimikrobiellen Wirkstoffe. Insofern ist das Backing bevorzugt frei von antimikrobiellen Wirkstoffen und ist bevorzugt auch nicht mit derartigen Wirkstoffen beschichtet oder behandelt.

Eine besonders bevorzugte Ausführungsform eines Wundkissens umfasst

a) eine erste Vliesschicht in distaler Position, welche dazu bestimmt ist, von der Wunde wegzuweisen, wenn das Wundkissen während der Wundbehandlung über der Wunde positioniert ist,
b) eine zweite Vliesschicht, wobei die zweite Vliesschicht vorzugsweise aus einem anderen Material oder einer Materialmischung als die erste Vliesschicht besteht und eine proximale Ausrichtung hat und somit dazu bestimmt ist, der Wunde zuzuweisen, wenn das Wundkissen während der Wundbehandlung über der Wunde positioniert ist,
c) eine Ultraschallschweißung, die einen Rand der ersten Vliesstoffschicht und einen Rand der zweiten Vliesstoffschicht miteinander verbindet, so dass die erste Vliesstoffschicht und die zweite Vliesstoffschicht eine Tasche bilden. Innerhalb der Tasche ist ein absorbierender Kern angeordnet. Vorzugsweise umfassen

die erste Vliesstoffschicht und die zweite Vliesstoffschicht Kunstfasern, die aus demselben Material bestehen, da dies die Qualität der Schweißnaht erhöht. Das Wundkissen ist dadurch gekennzeichnet, dass die zweite Vliesschicht auf ihrer proximalen Seite (Außenseite) die erfindungsmäße antimikrobielle Beschichtung trägt. Ein derart ausgestaltetes Wundkissen kann Teil der hierin beschriebenen Wundauflagen sein.

Bei den unter c) genannten Kunstfasern kann es sich um Fasern aus Polypropylen handeln, welche optional im Falle der zweiten (proximalen) Vliesschicht in einer Materialmischung mit Viskosefasern vorliegen können.

Weiterhin kann der erfindungsgemäße Vliesstoff - insbesondere, wenn er als Teil einer Wundauflage vorliegt - von einer oder mehreren zusätzlichen Schichten überdeckt werden. Beispielsweise kann der erfindungsgemäße Vliesstoff von einer distal gelegenen weiteren Schicht überlagert bzw. abgedeckt werden. Diese überlagernde Schicht kann Polypropylen enthalten. Bevorzugt enthält die überlagernde Schicht mindestens 90 Gew.-% oder mindestens 99 Gew.-% Polypropylen. Besonders bevorzugt besteht die überlagernde Schicht gänzlich aus Polypropylen. Das Polypropylen verleiht der überlagernden Schicht wasserabweisende Eigenschaften und schützt das darunter befindliche Material vor von außen einwirkenden Flüssigkeiten wie z.B. Wasser. Eine überlagernde Schicht enthaltend mindestens 90 Gew.-% Polypropylen ist damit eine wasserabweisende Schicht. Die Verdunstung von innen nach außen (z.B. ausgehend von absorbiertem Wundexsudat) bleibt im Wesentlichen erhalten. Das Flächengewicht der überlagernden Schicht kann beispielsweise 20 bis 30 g / m$^2$ betragen. Die überlagernde Schicht kann durch Schweißen im Randbereich am Vliesstoff (z.B. an der inneren Lage) befestigt werden. Durch das Schweißen entsteht eine Schweißnaht. Diese Schweißnaht ist bevorzugt durchgehend im Randbereich ausgebildet, so dass eine umlaufende Schweißnaht vorhanden ist und eine geschlossene Tasche entsteht, in deren Zentrum ein Hohlraum ausgebildet ist. Dieser Hohlraum der geschlossenen Tasche kann mit einer oder mehreren saugfähigen Materialien gefüllt sein. Bei diesen saugfähigen Materialien kann es sich um Folgendes handeln: zellulosehaltige Fasern oder zellulosehaltige Flocken, superabsorbierendes Polymer und ein Gemisch aus dem zuvor genannten. Das superabsorbierende Polymer kann Polyacrylsäure oder deren Derivate enthalten. Die Polyacyrylsäure oder deren Derivate können quervernetzt sein, um die Gefahr eines Zerfließens nach Flüssigkeitskontakt zu reduzieren. Das superabsorbierende Polymer kann in Form von Fasern und / oder Partikeln vorliegen.

Ein Vorteil das saugfähige Material in der geschlossenen Tasche unterzubringen, besteht darin, dass eine unerwünschte Wechselwirkung zwischen der superabsorbierenden Substanz, welche in aller Regel eine negative Ladung durch das Vorhandensein von Acrylsäure ausbildet, und dem positiv geladenen Polyarginin, Polylysin und / oder Polyornithin der erfindungsgemäßen Beschichtung vermieden wird. Gleichzeitig können die vorteilhaften Absorptionskapazitäten superabsorbierender Substanzen genutzt werden. Zudem hat das in der Tasche untergebrachte saugfähige Material polsternde Eigenschaften. Polsterndes Material trägt darüber hinaus dazu bei, die Kontaktfläche zwischen der antimikrobiellen Beschichtung und dem Wundgewebe zu maximieren, indem das polsternde saugfähige Material den beschichteten Vliesstoff sanft an das Wundgewebe andrückt. Die antimikrobielle Wirksamkeit wird dadurch gesteigert. Dieser Effekt ist besonders ausgeprägt bei Einsatz von quellfähigen saugfähigen Materialien wie superabsorbierendem Polymer.

Im Falle einer Wundauflage ist die letzte distale Schicht bevorzugt in Form eines Backings der Wundauflage ausgestaltet. Das Backing kann dabei mittels eines Adhäsivs (z.B. Acrylkleber) angebracht sein. Bevorzugt wird das Backing auf die Oberseite der geschlossenen Tasche aufgebracht. Wenn das mit einem Adhäsiv beschichtete Backing die darunter befindlichen Materialien der Wundauflage zu allen Seiten überragt, bildet es einen umlaufenden Kleberand aus, wodurch die Wundauflage die Form des bereits erwähnten Inselverbands erhält. Auf die Zusammensetzung des Backings wird an anderer Stelle eingegangen.

Eine Wundauflage kann den beschichteten Vliesstoff als Absorptionsschicht enthalten, welche gleichzeitig eine Wundkontaktschicht darstellt. Darüber hinaus kann eine Wundauflage den beschichteten Vliesstoff und eine zusätzliche Absorptionsschicht enthalten. Wenn der beschichtete Vliesstoff innerhalb einer Wundauflage in Kombination mit einer Absorptionsschicht vorliegt, kann der Vliesstoff bevorzugt weniger hydrophil sein als die Absorptionsschicht, so dass eine vom Vliesstoff aufgenommene Flüssigkeit weiter an die Absorptionsschicht geleitet wird, wo sie gespeichert wird. Bei der Absorptionsschicht kann es sich beispielsweise um einen Saugkörper handeln, der die oben erläuterte Tasche aufweist oder der Saugkörper kann beispielsweise als Wundkissen vorliegen oder Teil eines Wundkissens sein.

Wenn der erfindungsgemäße Vliesstoff mit einer zusätzlichen Absorptionsschicht innerhalb einer Wundauflage kombiniert wird, kann der Vliesstoff sowohl hydrophob als auch hydrophil sein. Bei alleiniger Verwendung als Wundpad in einem Pflaster kann der Vliesstoff bevorzugt hydrophil sein, da auf diese Weise eine schnellere Aufnahme von Flüssigkeit in den Vliesstoff und Verteilung der Flüssigkeit innerhalb des Vliesstoffes ermöglicht wird.

Bevorzugt umfasst die erfindungsgemäße Wundauflage eine Absorptionsschicht, welche auf der zweiten, distalen Seite des Vliesstoffes angebracht ist und / oder einen umlaufenden Kleberand zum Ankleben der Wundauflage an die Haut, welche die Wunde umgibt und / oder ein rückseitiges Backing. Die Absorptionsschicht kann Vliesgewebe, Superabsorber, Flockenzellstoff oder ein geschäumtes Material umfassen. Die Absorptionsschicht kann darüber hinaus als Saugkörper ausgestaltet sein oder vom Saugkörper enthalten sein. Darüber hinaus kann die Absorptionsschicht Teil eines Wundkissens sein. Das Backing kann als Folie ausgebildet sein, die optional durchlässig für Wasserdampf, aber nicht für Flüssigkeiten wie Wasser, Blut oder Wundexsudat ist und die Polyurethan (PU) enthalten oder aus Polyurethan bestehen kann. Der umlaufende Kleberand kann Teil des Backings sein. Eine Absorptionsschicht bietet sich vor allem für blutende oder stark exsudierende Wunden an. Der Vorteil beim Vorhandensein eines Backings liegt darin, dass Wunde und Vliesstoff vor Nässe von außen geschützt werden, absorbierte Flüssigkeit nicht austreten kann und die übrigen Komponenten der Wundauflage vor dem Eindringen von Bakterien aus der Umgebung geschützt werden.

Die erfindungsgemäße Beschichtung des Vliesstoffes ist sterilisationsbeständig und behält ihre funktionellen und strukturellen Eigenschaften nach der Sterilisation bei. Insbesondere bleiben die antimikrobiellen und atraumatischen Eigenschaften erhalten. Weiterhin bleiben die strukturellen Eigenschaften erhalten, so dass sich die Beschichtung nicht löst oder zerfließt. Die Sterilisation kann dabei beispielsweise mittels Ethylenoxid, Dampfsterilisation (Autoklavierung) oder Heißluftsterilisation erfolgen. Bevorzugt ist der gesamte Vliesstoff inklusive Beschichtung sterilisationsbeständig. Dies kann beispielsweise dadurch erreicht werden, dass der Vliesstoff aus solchen Fasern oder Materialien aufgebaut ist, wie sie an anderer Stelle hierin beschrieben sind.

Die Sterilisationsbeständigkeit ist eine bevorzugte Eigenschaft von Wundversorgungsprodukten, da von Materialien, die in direktem Kontakt mit der Wunde kommen, oft Keimfreiheit gefordert wird.

Der Vliesstoff kann neben der erfindungsgemäßen antimikrobiellen Beschichtung weitere Verbindungen oder Strukturelemente enthalten. Hierzu gehören insbesondere Substanzen, die die Heilung fördern, die Wundränder vor Mazeration schützen und/oder die antimikrobielle Wirkung verbessern. Diese Substanzen können in die Beschichtung eingearbeitet, auf die proximale Seite der Beschichtung aufgetragen oder Teil des Vliesstoffes oder Teil einer Wundauflage mit besagtem Vliesstoff sein. Beispiele für Substanzen mit wundheilungsfördernder Wirkung sind Allantoin oder Dexpanthenol. Ein Beispiel für Substanzen mit antimikrobieller Wirkung sind Silber (kationisch oder elementar) und PHMB (Polyhexamethylenbiguanid).

Bevorzugt ist der beschichtete Vliesstoff oder die ihn enthaltende Wundauflage frei von pharmazeutischen Wirkstoffen, wie beispielsweise Hormonen, Schmerzmitteln etc. Es ist nicht ausgeschlossen, dass die antimikrobielle Beschichtung die Abgabe und / oder Aufnahme von Pharmazeutika beeinträchtigen oder beeinflussen könnte. In diesem Sinne können transdermale Produkte, wie beispielsweise transdermale Pflaster, von der hierin beschriebenen Erfindung ausgenommen sein.

Im Rahmen der vorliegenden Erfindung wird davon ausgegangen, dass die in der antimikrobiellen Beschichtung enthaltenen Inhaltsstoffe nicht in die Blutbahn des Patienten übergehen, da es sich um einen Kontaktmechanismus handelt.

Darüber hinaus umfasst die Erfindung auch ein Kit umfassend

a) einen erfindungsgemäßen beschichteten Vliesstoff oder eine Wundauflage, die besagten Vliesstoff enthält und
b) ein Befestigungsmittel, wobei das Befestigungsmittel geeignet ist zur Befestigung des Vliesstoffes oder einer Wundauflage mit besagtem Vliesstoff an einer Wunde.

Bei dem Befestigungsmittel kann es sich um eine Klebefolie handeln. Bei dem Kit kann es sich um eine Verpackung oder ein Set handeln.

Daneben betrifft die Erfindung auch die Verwendung des erfindungsgemäßen Vliesstoffes zur Herstellung der oben beschriebenen Wundauflage, die Verwendung des erfindungsgemäßen Vliesstoffes als Wundkontaktschicht in einer antimikrobiellen Wundauflage, die Verwendung des erfindungsgemäßen Vliesstoffes in einem Verfahren zur Herstellung einer antimikrobiellen Wundauflage sowie die Verwendung der beschriebenen antimikrobiellen Beschichtung in einem Verfahren zur Herstellung des erfindungsgemäßen Vliesstoffes.

Ein weiterer Aspekt der Erfindung betrifft den erfindungsgemäßen Vliesstoff oder eine Wundauflage, die diesen Vliesstoff umfasst, zur Anwendung in einem Verfahren zur Behandlung von Wunden, bevorzugt von infizierten Wunden, besonders bevorzugt von Wunden, die mit *S. aureus* und / oder *P. aeruginosa* infiziert sind.

Ein weiterer Aspekt der Erfindung betrifft die antimikrobielle Beschichtung zur Anwendung in einem Verfahren zur Behandlung von Wunden, bevorzugt von infizierten Wunden, wobei die Beschichtung auf einem Vliesstoff vorliegt.

Bei diesen beiden Anwendungsfällen kann vorgesehen sein, dass die Anwendung über einen Zeitraum von 0,1 bis 24 Stunden stattfindet oder dass die Anwendung in einem Zeitraum von mindestens 24 Stunden stattfindet, wobei letzteres beispielswiese bei bereits stark ausgeprägten Infektionen oder bei Patienten mit Immunschwäche geboten sein kann.

Der erfindungsgemäße Vliesstoff sowie eine Wundauflage mit besagtem Vliesstoff sind geeignet zur Abdeckung und / oder Therapie von Wunden, insbesondere infizierten Wunden. Aus diesen Gründen können Vliesstoff und Wundauflage weiterhin eingesetzt oder verwendet werden in einem Verfahren zur Wundtherapie und / oder zur Reduktion der Keimzahl in Wunden und / oder zur Prävention von Wundinfektionen. Bei den Wunden kann es sich insbesondere um Ulzera, traumatische Wunden (inklusive Schnitt- und Operationswunden), chronische Wunden, blutende Wunden, eiternde Wunden, nekrotische Wunden, belegte (fibrinhaltige) Wunden und exsudierende (nässende) Wunden handeln. Besonders vorteilhaft ist, dass der erfindungsgemäße Vliesstoff auch im Rahmen der Kompressionstherapie unter Kompressionsbandagen oder Kompressionsstrümpfen getragen werden kann.

Ferner von der Erfindung umfasst sind Verfahren zur Herstellung des hierin beschriebenen beschichteten Vliesstoffes, zum Auftragen der Beschichtung auf den Vliesstoff und zur Herstellung einer Wundauflage enthaltend den beschichteten Vliesstoff.

Nachfolgend wird ein Verfahren beschrieben, mit dem man einen Vliesstoff mit durchmischten Beschichtungen und / oder mit mindestens einer durchmischten Lage innerhalb der Beschichtung erzeugen kann:

> i) Bereitstellung eines Vliesstoffes, vorzugsweise eines Vliesstoffes zur Wundbehandlung
> ii) Besprühen des Vliesstoffes mit einer Hyaluronsäure und einem Polypeptid oder Polypeptiden zur Ausbildung einer Beschichtung auf dem Vliesstoff umfassend mindestens eine antimikrobielle Beschichtungslage,
> iii) optionale Rücktrocknung des beschichteten Vliesstoffes

wobei es sich bei dem Polypeptid oder den Polypeptiden um Polyarginin und / oder Polylysin und / oder Polyornithin handelt und wobei die mindestens eine Lage sowohl die Hyaluronsäure als auch das Polypeptid oder die Polypeptide enthält.

Das Besprühen kann beispielsweise mittels eines Zerstäubers oder einer Sprühpistole geschehen. Bevorzugt handelt es sich um ein elektrisches Sprühgerät, das eine konstante Menge an Flüssigkeitsvolumen pro Zeiteinheit versprüht.

Einer der großen Vorteile dieses Verfahrens ist die Zeitersparnis. In diesem Sinne kann vorgesehen sein, dass das Aufsprühen einer Lage maximal 5 min, bevorzugt maximal 1 min, besonders bevorzugt maximal 30 s und ganz besonders bevorzugt maximal 5 s dauert. Die Lage kann dabei eine Fläche von 10 cm x 10 cm haben.

Weiterhin kann vorgesehen sein, dass der gesamte Beschichtungsprozess mittels Aufsprühen vom Beginn des Sprühvorgangs bis zum Erhalt des fertigen Vliesstoffes maximal 30 min dauert, wobei die Trocknung des Vliesstoffes in diesem Zeitraum bereits inkludiert sein kann. Der Vliesstoff kann dabei eine Fläche von 10 cm x 10 cm haben.

Bevorzugt liegen die Hyaluronsäure und das Polypeptid oder die Polypeptide während des Besprühens gemäß Schritt ii) in Lösung vor. Mögliche Lösungen wie polare Flüssigkeiten, insbesondere Wasser und wässrige Pufferlösungen wurden oben bereits beschrieben und können im Rahmen des zuletzt genannten Verfahrens eingesetzt werden.

Bevorzugt beträgt die Konzentration des in Lösung vorliegenden Polypeptids oder der Polypeptide während des Besprühens gemäß Schritt ii) bei 0,1 mg / ml bis 100 mg / ml, besonders bevorzugt bei 1 mg / ml bis 80 mg / ml, ganz besonders bevorzugt bei 3 mg / ml bis 50 mg / ml und am besten 5 mg / ml bis 30 mg / ml. Bevorzugt beträgt die Konzentration der Hyaluronsäure während des Besprühens gemäß Schritt ii) bei 0,1 mg / ml bis 10 mg / ml, besonders bevorzugt 0,5 mg / ml bis 8 mg /ml, ganz besonders bevorzugt bei 1 mg / ml bis 5 mg / ml und am besten 2 mg / ml bis 4 mg / ml.

Bevorzugt werden durch das Besprühen gemäß Schritt ii) 0,1 bis 1 ml einer solchen Lösung des Polypeptids bzw. der Polypeptide und / oder 0,1 ml bis 1 ml einer solchen Lösung der Hyaluronsäure pro $cm^2$ der proximalen (wundzu-

gewandten) Seite des Vliesstoffes aufgesprüht. Besonders bevorzugt werden 0,2 bis 0,9 ml, ganz besonders bevorzugt 0,3 bis 0,8 ml und am besten 0,4 bis 0,7 ml einer solchen Lösung aufgesprüht. Die Volumenangaben sind pro Lage zu verstehen. Da im Falle durchmischter Lagen bereits eine einzige Lage eine fertige Beschichtung ausbilden kann, können die Volumenangaben in einem solchen Fall auch pro Beschichtung (enthaltend eine Lage) verstanden werden.

Gemäß einem weiteren Aspekt der Erfindung wird bei dem oben beschriebenen Verfahren das Besprühen gemäß Schritt ii) mindestens zwei Mal ausgeführt, um eine Beschichtung mit mindestens zwei Lagen zu erzeugen. Zwischen dem Besprühen kann eine Trocknungsphase liegen. Diese kann 30 s bis 30 min betragen, bevorzugt 1 min bis 25 min, ganz besonders bevorzugt 2 min bis 20 min und am besten 3 min bis 15 min.

Teil der Erfindung ist auch ein Vliesstoff wie hierin beschrieben, erhältlich oder erhalten durch das zuletzt erläuterte Herstellungsverfahren.

Ein anderes erfindungsgemäßes Verfahren zur Herstellung eines beschichteten Vliesstoffes umfasst die folgenden Schritte:

i) Bereitstellung eines Vliesstoffes

ii) Beschichten des Vliesstoffes durch Auftragen von mindestens zwei übereinanderliegenden Lagen, wobei mindestens eine Lage ein Polypeptid oder Polypeptide enthält, ausgewählt aus Polyarginin, Polylysin und Polyornithin oder einer Mischung aus mindestens zwei der zuvor genannten Polypeptide, und eine positive Nettoladung hat und mindestens eine Lage eine Hyaluronsäure enthält und eine negative Nettoladung hat und wobei die übereinanderliegenden Lagen in ihrer Nettoladung alternieren

iii) optionale ein- oder mehrmalige Wiederholung von Schritt ii)

iv) optionales Rücktrocknen des beschichteten Vliesstoffes.

Der Beschichtungsschritt ii) kann insbesondere mittels Tauchverfahren ausgeführt werden. Andere Beschichtungstechniken sind ebenfalls möglich. So kann das Beschichten beispielsweise alternativ auch durch Auftragung mittels Walze oder durch Bestreichen (z.B. mittels Pinsel) erfolgen.

Empfohlen wird sowohl die Hyaluronsäure als auch das Polypeptid oder die Polypeptide vor dem Beschichten in Lösung zu bringen. Geeignete Lösungsmittel und Konzentrationen wurden hierin bereits an anderer Stelle beschrieben und sind im Rahmen des zuletzt genannten Verfahrens anwendbar. So können das Polypeptid oder die Polypeptide beispielsweise mittels einer Lösung aufgebracht werden, in der sie in einer Konzentration von 0,1 mg / ml bis 100 mg / ml enthalten sind und / oder beispielsweise die Hyaluronsäure mittels einer Lösung aufgetragen werden, die die Hyaluronsäure in einer Konzentration von 0,1 bis 10 mg / ml enthält.

Dieses Verfahren eignet sich insbesondere zur Herstellung solcher Vliesstoffe, deren Beschichtung mindestens zwei nicht durchmischte Lagen enthält. Nicht durchmischte Lagen sind weiter oben in ihrer Beschaffenheit erläutert. In der Regel enthalten die mit dem obigen Verfahren erzeugten Beschichtungen mindestens zwei Lagen. Bevorzugt sind sämtliche Lagen der mittels dieses Verfahrens erzeugten Beschichtung nicht durchmischt.

Im Rahmen des zuletzt genannten Verfahrens ist es möglich eine Vielzahl von Lagen zu einer Beschichtung zu vereinen. Empfohlen wird im Rahmen von Schritt ii) nach dem Auftragen einer Lage diese mit einer Pufferlösung zu spülen. Dies sollte erst dann geschehen, wenn die zu spülende Lage ausreichend getrocknet oder angetrocknet ist, um ein ungewolltes Abwaschen zu vermeiden.

Geeignete Pufferlösungen und pH-Werte sind hierin bereits an anderer Stelle genannt worden. Bevorzugt wird hierzu wässrige Pufferlösung von Tris-NaCl verwendet. Besonders bevorzugt sind sowohl Tris als auch NaCL in einer Konzentration von jeweils 10 mmol bis 150 mmol in der Pufferlösung vorhanden. Die Konzentrationen von Tris und NaCl müssen dabei nicht identisch sein.

Weiterhin kann das Beschichten gemäß Schritt ii) des zuletzt genannten Verfahrens durch (vollständiges oder teilweises) Eintauchen des Vliesstoffes in eine Lösung, die das Polypeptid oder die Polypeptide enthält und / oder in eine Lösung, die die Hyaluronsäure enthält, erfolgen.

Das (vollständige oder teilweise) Eintauchen kann dabei für die Erzeugung einer einzelnen Lage über eine Zeitspanne von ein bis zehn Minuten erfolgen. Das heißt, dass der Vliesstoff in die Lösung eingetaucht wird und nach ein bis zehn Minuten entnommen wird. Die folgenden alternativen Zeitspannen sind ebenfalls möglich: zwei bis neun Minuten, drei bis acht Minuten sowie vier bis sieben Minuten.

Teil des zuletzt genannten Verfahrens ist darüber hinaus, dass Schritt ii) wiederholt stattfinden kann (also mindestens zwei Mal). So kann Schritt ii) für die Herstellung eines erfindungsgemäßen Vliesstoffes beispielsweise zehn bis hundert Mal wiederholt werden, so dass 20 bis 200 Lagen übereinanderliegend aufgetragen werden. Dies resultiert folglich in einem Vliesstoff mit einer Beschichtung, die 20 bis 200 Lagen enthält. Andere mögliche Wiederholungsanzahlen für Schritt ii) sind 15 bis 90 Mal, 20 bis 80 Mal, 25 bis 70 Mal und 30 bis 60 Mal.

Wenn die Beschichtungsauftragung gemäß Schritt ii) mittels Tauchverfahren ausgeführt wird, beziehen sich die eben genannten Wiederholungszahlen auf die Anzahl des Eintauchens in eine Lösung enthaltend die Hyaluronsäure oder das Polypeptid bzw. die Polypeptide.

Teil der Erfindung ist auch ein beschichteter Vliesstoff wie hierin beschrieben, erhältlich oder erhalten durch das zuletzt erläuterte Herstellungsverfahren.

Die hierin beschriebenen Herstellungsverfahren können sich bei Bedarf durch folgende Punkte auszeichnen:

- Es kann eine Seite oder es können beide Seiten des Vliesstoffes (proximale und distale Oberfläche) beschichtet werden. Wenn beide Seiten beschichtet werden, so kann dies nacheinander oder gleichzeitig erfolgen.
- Auf einen Vliesstoff mit negativer Nettoladung oder negativer Außenladung (negative Ladung der Oberfläche) kann zuerst eine Lage enthaltend das Polypeptid oder die Polypeptide als erste Lage aufgetragen werden. In diesem Fall hat die erste Lage eine positive Nettoladung. Bevorzugt enthält in diesem Fall die erste Lage keine Hyaluronsäure.
- Auf einen Vliesstoff mit positiver Nettoladung oder positiver Außenladung (positive Ladung der Oberfläche) kann zunächst eine Lage enthaltend die Hyaluronsäure als erste Lage aufgetragen werden. In diesem Fall hat die erste Lage eine negative Nettoladung. Bevorzugt enthält in diesem Fall die erste Lage kein Polypeptid bzw. keine Polypeptide.

Ein weiterer Teil der Erfindung betrifft die hierin beschriebenen Herstellungsverfahren, wobei der bereitgestellte Vliesstoff zunächst einer Plasmareinigung unterzogen wird. Die Plasmareinigung findet vor der Beschichtung, Auftragung oder dem Besprühen statt und erlaubt ein noch besseres Anhaften der späteren Beschichtung auf dem Vliesstoff.

Weiterhin können die hierin beschriebenen Herstellungsverfahren automatisiert sein. Bevorzugt findet die Automatisierung mittels eines Roboters statt. Bei dem Roboter kann es sich um einen programmierbaren Roboter handeln. Der Roboter kann über mindestens einen beweglichen Arm verfügen, an dem ein oder mehrere Vliesstoffe angebracht werden können. Insbesondere das Auftragen der Beschichtung mittels Tauchverfahren profitiert von einer Automatisierung, da diese Methode in der Regel mehr Zeit beansprucht als das Auftragen der Beschichtung mittels Sprühverfahren. Bevorzugt werden bei dem automatisierten Tauchverfahren Beschichtungen mit mindestens 20, besser 25 und am besten 30 Lagen auf dem Vliesstoff erzeugt. Wenn das erfindungsgemäße Herstellungsverfahren mittels eines Roboters stattfindet, können mehrere Vliesstoffe gleichzeitig beschichtet werden. Beispielsweise können zwei, mindestens zwei, drei, mindestens drei, vier oder mindestens vier, fünf oder mindestens fünf Vliesstoffe gleichzeitig auf diese Weise beschichtet werden. Besonders bevorzugt werden zwei bis hundert, zwei bis fünfzig oder zwei bis zehn Vliesstoffe gleichzeitig unter Einsatz eines einzigen Roboters zeitgleich beschichtet.

Das Sprühverfahren profitiert ebenfalls von einer Automatisierung, da hierdurch ein gleichbleibender Abstand zwischen Sprühdüse und Vliesstoff während des Sprühvorgangs gewährleistet wird, was bei einem händischen Ansatz deutlich schwieriger umzusetzen ist.

Figuren

Im Folgenden werden die Figuren näher erläutert. Wo Keimzahlen dargestellt sind, sind diese anhand eines dekadisch-logarithmischen Maßstabs der Ordinate wiedergegeben.

Fig. 1 zeigt eine Fluoreszenzaufnahme eines tauchbeschichteten Vliesstoffes mittels Konfokalmikroskop. Die Beschichtung enthält 24 Doppellagen aus Polyarginin und Hyaluronsäure, wobei das in der Beschichtung enthaltene Polyarginin mittels des fluoreszierenden Markers FITC sichtbar gemacht wurde.

Fig. 2 zeigt eine Fluoreszenzaufnahme eines sprühbeschichteten Vliesstoffes mittels Konfokalmikroskop. Die Beschichtung enthält eine durchmischte Lage aus Polylysin und Hyaluronsäure, wobei das in der Beschichtung enthaltene Polylysin mittels des fluoreszierenden Markers FITC sichtbar gemacht wurde.

Fig. 3a zeigt die antimikrobielle Wirksamkeit von Vliesstoffen mit einer polyargininhaltigen Tauchbeschichtung (48 alternierende Einzelschichten aus entweder Polyarginin oder Hyaluronsäure; entsprechend 24 Doppelschichten) gegenüber *P. aeruginosa* nach Initialkontakt (t = 0h) sowie einen Tag später (t = 24 h). Der Test erfolgte nach ISO 20743:2021.

Fig. 3b zeigt Werte für die gleiche Anordnung wie für Fig. 3a beschrieben, allerdings in diesem Fall gegenüber *S. aureus.*

Fig. 4a zeigt die antimikrobielle Wirksamkeit von Vliesstoffen mit einer polylysinhaltigen Tauchbeschichtung (48 alternierende Einzelschichten aus entweder Polylysin oder Hyaluronsäure; entsprechend 24 Doppelschichten) gegenüber *P. aeruginosa* nach einer Wirkungsdauer von einem Tag. Der Test erfolgte nach ISO 20743:2021.

Fig. 4b zeigt Werte für die gleich Anordnung wie Fig. 4a beschrieben, allerdings in diesem Fall gegenüber *S. aureus.*

Fig. 5a zeigt den Vergleich der antimikrobiellen Wirksamkeit von tauchbeschichteten Vliesstoffen mit Polyarginin, Polylysin sowie einer Mischung dieser beiden Polypeptide (24 Doppelschichten aus entweder Polyarginin oder Polylysin oder einer Mischung aus diesen beiden Polypeptiden jeweils zusammen mit Hyaluronsäure) gegenüber *P. aeruginosa* nach einer Wirkungsdauer von einem Tag. Dargestellt sind die Mittelwerte aus einer dreifachen biologischen und dreifachen technischen Wiederholung. Der Test erfolgte nach ISO 20743:2021.

Fig. 5b zeigt Werte für die gleiche Anordnung wie für Fig. 5a beschrieben, allerdings in diesem Fall gegenüber *S. aureus.*

Fig. 6a zeigt die antimikrobielle Wirksamkeit von Vliesstoffen mit einer polylysinhaltigen Sprühbeschichtung (eine durchmischte Lage enthaltend Polylysin und Hyaluronsäure) gegenüber *P. aeruginosa* nach einer Wirkungsdauer von einem Tag. Dargestellt sind die Mittelwerte aus drei Wiederholungen. Der Test erfolgte nach ISO 20743:2021.

Fig. 6b zeigt Werte für die gleiche Anordnung wie für Fig. 6a beschrieben, allerdings in diesem Fall gegenüber *S. aureus.*

Fig. 7 zeigt die antimikrobielle Wirksamkeit von sprühbeschichteten Vliesstoffen mit zwei unterschiedlichen Konzentrationen an Polyarginin. Der Test erfolgte gegenüber *P. aeruginosa* gemäß ISO 20743:2021. Die eingesetzten Konzentrationen waren: eine durchmischte Lage aus 1 mg / mL Polyarginin + 1 mg / mL Hyaluronsäure sowie eine durchmischte Lage aus 2 mg / mL Polyarginin + 1 mg / mL Hyaluronsäure.

Fig. 8 zeigt die Werte aus Fig. 7 im Vergleich zur antimikrobiellen Wirksamkeit, die eine handelsübliche silberhaltige Wundauflage unter gleichen Bedingungen erzielte.

Fig. 9a zeigt die antimikrobielle Wirksamkeit von simultan mittels eines Roboters tauchbeschichteten Vliesstoffen (48 alternierende Einzellagen aus jeweils Polyarginin oder Hyaluronsäure; entsprechend 24 Doppellagen) gegenüber *P. aeruginosa.*

Fig. 9b zeigt Werte für die gleiche Anordnung wie für Fig. 9a beschrieben, allerdings in diesem Fall gegenüber *S. aureus.*

**Beispiele**

**Beispiel 1: Materialien und Vliesstoffe**

An Materialien wurden bereitgestellt:

- Polypeptide vom Typ Polyarginin als synthetisches Polymer bestehend aus 30 Aminosäuren pro Molekül ("PAR30"), so dass jedes Molekül eine Molekülmasse von etwa 5,8 kDa hatte. Das Polyarginin wurde vom Unternehmen "AlamandaTM Polymers" bezogen. Die eingesetzte Konzentration lag bei 0,5 mg / ml.

- Polypeptide vom Typ Polylysin, welche als ε-Poly(L-Lysin) natürlichen Ursprungs (Herstellung mittels Bakterien aus der Familie der *Streptomycetaceae*) vom Unternehmen Biosynth® bezogen wurden. Die mittlere Molekülmasse lag zwischen 3,5 und 4,5 kDa. Die eingesetzte Konzentration lag bei 10 mg / ml Tris-NaCl-Puffer.

- Hyaluronsäure aus 144 repetitiven Untereinheiten pro Molekül ("HA144"). Hergestellt mittels rekombinanter Produktion aus Mikroorganismen. Die eingesetzte Konzentration lag bei 0,5 mg / ml in Tris-NaCl-Puffer. Bezogen wurde die Hyaluronsäure vom Unternehmen Lifecore® Biomedical.

An Vliesstoffen wurden bereitgestellt:

- Vliesstoff A: Ein aus zwei miteinander verbundenen Lagen bestehender Vliesstoff. In proximaler Richtung (in Benutzung wundseitig) eine äußere Vliesschicht aus Polypropylen mit einem Flächengewicht von 18 g / m². In distaler Richtung (in Benutzung von der Wunde wegzeigend) eine innere Vliesschicht aus 60 Gew.-% Viskose und 40 Gew.-% Polypropylen mit einem Flächengewicht von 27 g / m². Das obere und das untere Vlies wurden thermisch zum Vliesstoff A verbunden, welcher ein Gesamtflächengewicht von 45 g / m² hatte und zu 64 Gew.-% Polypropylen und zu 36 Gew.-% aus Viskose bestand.

- Vliesstoff B: Vliesstoff aus Polyamid und Viskose mit einem Flächengewicht von 37 g/m$^2$.

- Vliesstoff C: Vliesstoff aus 60 Gew.-% Viskose und 40 Gew.-% Polyester mit einem Flächengewicht von 40 g/m$^2$

Die aufgeführten Vliesstoffe A bis C waren allesamt geeignet, um mit der antimikrobiellen Beschichtung ausgestattet zu werden. Gleichzeitig handelte es sich um Textilmaterialien, die die Erfordernisse zur Wundversorgung erfüllten.

**Beispiel 2: Beschichtung mittels Tauchverfahren**

Als Ausgangsmaterial diente Vliesstoff A wie unter Beispiel 1 beschrieben.

Der Vliesstoff wurde auf eine Fläche von 2 cm x 2 cm zugeschnitten und zunächst in einem Autoklaven sterilisiert. Während des nachfolgenden Beschichtungsvorgangs mittels Tauchverfahren wurde der Vliesstoff abwechselnd in ein Bad mit Polyarginin ("PAR30" ; 0,5 mg / mL) und ein Bad mit Hyaluronsäure (0,5 mg / mL) eingetaucht. Der erste Eintauchschritt erfolgte dabei in der Lösung mit Polyarginin. Das Eintauchen erfolgte jeweils für 200 Sekunden. Nach jedem Tauchschritt wurde das Substrat gespült, und zwar ebenfalls für 200 Sekunden. Hierzu wurde Tris-NaCl-Puffer verwendet (10 mmol Tris, 150 mmol NaCl, pH 7,4). Der Prozess fand voll automatisiert statt unter Einsatz eines Roboters der Firma Riegler & Kirstein GmbH.

Der Programmablauf des Roboters sah wie folgt aus:

A6+(A5+A6+B5+B6+*A4*+A3+B4+B3)*8+(A5+A6+A7+B7+*A4*+A3+A2+B2)*8+(A5+A6+A8+B8+*A4*+A3+A1+B1)*8+eB1

Legende:

A, B = Positionen des Roboterarms
unterstrichen = Auftrag des Polypeptids
kursiv = Auftrag der Hyaluronsäure
Programmdauer: ca. 13h

Die genannten Schritte wurden so oft wiederholt, bis 24 Doppellagen auf das Substrat aufgetragen waren. Jede Doppellage bestand aus einer Lage enthaltend das Polyarginin und einer Lage enthaltend die Hyaluronsäure. Somit enthielt die fertige Beschichtung 48 Einzellagen. Der Beschichtungsvorgang dauerte insgesamt etwa 13 Stunden. Bei Bedarf kann der Prozess auch auf eine Dauer von unter zwei Stunden verkürzt werden. Nach einer Trocknung (über Nacht, passiv bei Raumtemperatur) erfolgte im Anschluss eine weitere Sterilisation mittels U.V.-Bestrahlung, und zwar über einen Zeitraum von mindestens 30 min für jeweils die proximale und distale Oberfläche der Wundkontaktschichten.

Die beschichteten Vliesstoffe wurden anschließend in einem geschlossenen Raum über mehrere Wochen gelagert.

**Beispiel 3a: Auftrag einer polylysinhaltigen Beschichtung mittels Sprühverfahren**

Als Ausgangsmaterial diente Vliesstoff A wie unter Beispiel 1 beschrieben. Es wurden vier Prüfmuster mit einer Fläche von jeweils 2,25 cm$^2$ bereitgestellt. Bei der Beschichtung mittels Sprühverfahren kamen eine Lösung enthaltend ε-Poly(L-Lysin) (10 mg / ml) und eine Lösung enthaltend Hyaluronsäure ("HA144" ; 0,5 mg / ml) zum Einsatz. Als Lösungsmittel diente Tris-NaCl-Puffer. Beide Lösungen wurden zeitgleich über jeweils eine separate Düse einer Sprühpistole auf das Vliesmaterial aufgesprüht. Das Aufsprühen fand aus einer Distanz von 10 bis 15 cm statt. Gesprüht wurde auf diejenige Seite der Prüfmuster, welche aus 100% Polypropylen bestand (vgl. Bsp. 1). Dabei wurde die Sprühpistole während des Sprühens zehn Mal für jeweils eine Sekunde über jedes Prüfmuster geführt, um pro Muster insgesamt 1,5 ml von jeder Lösung aufzutragen. Somit Betrug das Flächenvolumen der Beschichtungslösung 0,66 ml / cm$^2$ Vliesoberfläche. Der Beschichtungsvorgang dauerte 10 - 15 Sekunden. Anschließend folgte eine Trocknung (über Nacht, passiv bei Raumtemperatur).

**Beispiel 3b: Auftrag einer polyargininhaltigen Beschichtung mittels Sprühverfahren**

Das Vorgehen war analog zu Beispiel 3a. Abweichend von den unter Beispiel 1 angegebenen Konzentrationen kamen hierbei folgende Lösungen zum Einsatz: a) 1 mg / ml Polyarginin in Kombination mit 1 mg / ml Hyaluronsäure sowie b) 2 mg / ml Polyarginin in Kombination mit 1 mg / ml Hyaluronsäure.

**Beispiel 4a: Visuelle Inspektion der Tauchbeschichtung mittels Konfokalmikroskop**

Die im Tauchverfahren gemäß Beispiel 2 erzeugte Beschichtung wurde einer visuellen Prüfung mittels Konfokalmikroskop unterzogen. Hierzu wurde das in der Beschichtung enthaltene Polyarginin mit dem Fluorophor Fluoresceinisothiocyanat (FITC) markiert (PAR30-FITC).

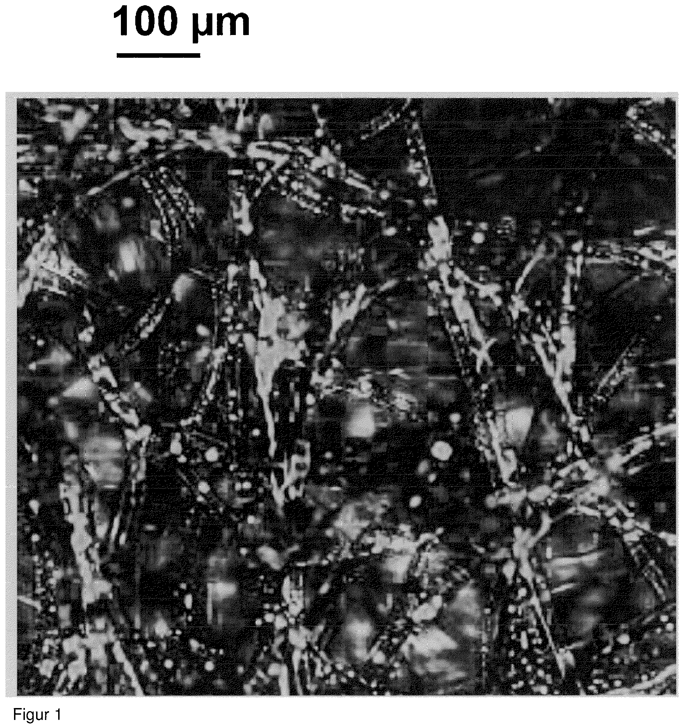

Die Verteilung der auf diese Weise zur Fluoreszenz gebrachten Moleküle wurde mit einem Mikroskop vom Typ Zeiss LSM 710 (Konfokales Laserscanning Mikroskop) begutachtet. Dies fand unter einer 40-fachen Vergrößerung statt. Die korrekte Anhaftung der Beschichtung an der Faserstruktur des verwendeten Vliesstoffes konnte verifiziert werden. Das Ergebnis ist in Fig. 1 als Fotoaufnahme dargestellt.

Wie zu erkennen ist, umschließt die Beschichtung die Faserstruktur des Vliesstoffes zu allen Seiten auf gleichmäßige Weise. Dies lässt auf eine erfolgreiche Anhaftung der Beschichtung am Substrat schließen. Eine unbeschichtete Kontrollaufnahme eines ansonsten identischen Vliesstoffes zeigte keinerlei Fluoreszenz (nicht abgebildet).

**Beispiel 4b: Visuelle Inspektion der Sprühbeschichtung mittels Konfokalmikroskop**

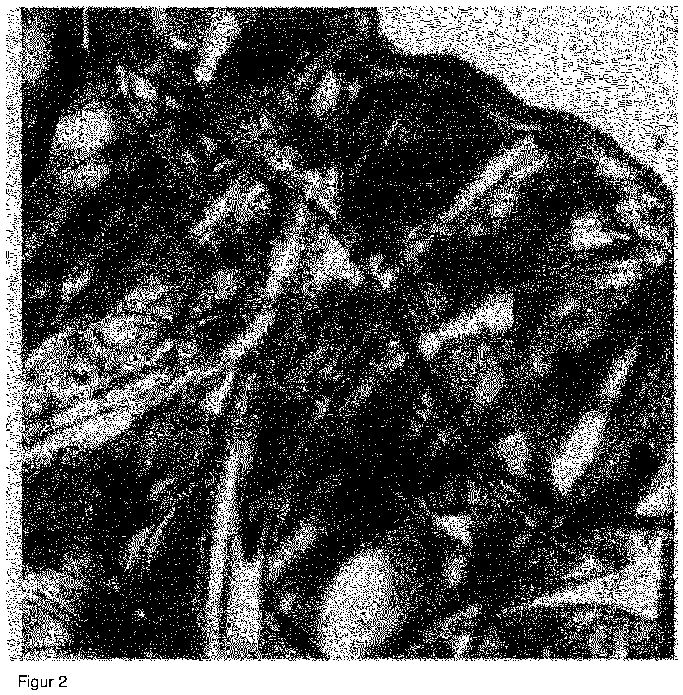

Nach dem gleichen Vorgehen wie in Beispiel 4a wurde eine Fluoreszenzaufnahme des mittels Sprühverfahren beschichteten Vliesstoffes aus Beispiel 3 angefertigt. Das Ergebnis ist in Fig. 2 dargestellt. Auch in diesem Fall konnte die erfolgreiche Anhaftung der Beschichtung an den Fasern nachgewiesen werden.

**Beispiel 5a: Antimikrobielle Wirksamkeit einer polyargininhaltigen Tauchbeschichtung**

Die Tests zur antimikrobiellen Wirksamkeit wurden an den in Beispiel 2 mittels Tauchverfahren erzeugten beschichteten Vliesstoffen durchgeführt, wobei sowohl gramnegative als auch grampositive Bakterienstämme zum Einsatz kamen. Als gramnegative Kultur diente ein Stamm von *Pseudomonas aeruginosa* (aus Hinterlegung ATTC 27853) und als grampositive Kultur ein Stamm von *Staphylococcus aureus* (aus Hinterlegung ATTC 25923).

Als Testprotokoll diente der Standard ISO 20743:2021 (Owen L, Laird K. Development of a silver-based dual-function antimicrobial laundry additive and textile coating for the decontamination of healthcare laundry. J Appl Microbiol. 2021 ;130(4):1012-22). Beim Test wurde die Reduktion der Anzahl teilungsfähiger Bakterienzellen (CFU) auf den textilen Oberflächen nach einer Kontaktzeit von 0 h und 24 h ermittelt. Die antimikrobielle Beschichtung war zuvor im Tauchverfahren auf die Textilien aufgetragen worden und bestand aus einer alternierenden Abfolge von PAR30 und HA144 mit 48 Einzellagen. Weitere Details können Beispiel 2 entnommen werden.

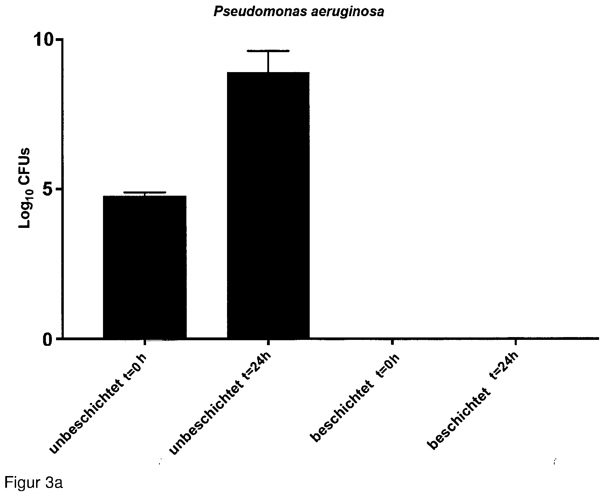

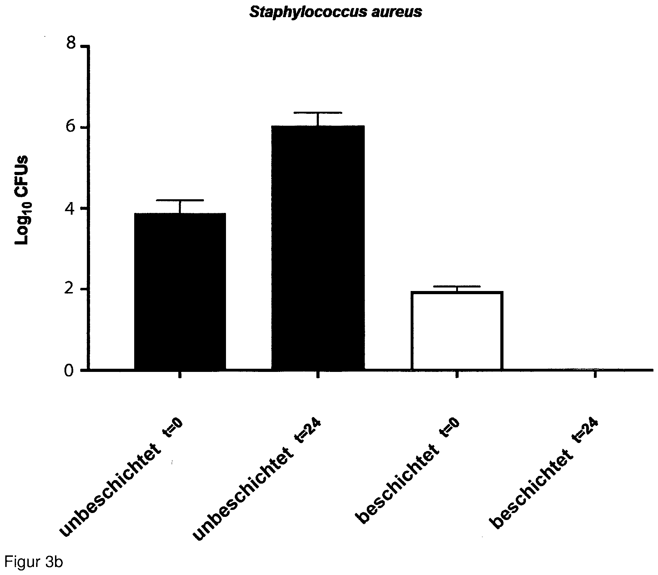

Zunächst wurden die beschichteten Vliesstoffe mit einer Bakterienausgangskonzentration von 1 bis $3 \times 10^5$ CFU / ml beimpft und bei 37 °C inkubiert. Im Anschluss (nach 0 bzw. 24 h) wurden die überlebenden Bakterien in PBS eluiert. Das Eluat wurde abwechselnd gevortext (mit hoher Frequenz mittels eines Vortex Gerätes geschüttelt), sonifiziert und wieder gevortext (jeweils 30 Sek. in dreifacher Wiederholung). Die Proben wurden sowohl im trockenen als auch im feuchten Zustand (nach Zugabe von PBS) getestet, um ein feuchtes Wundmilieu zu simulieren. Als Kontrolle dienten unbeschichtete Vliesstoffe mit ansonsten identischem Aufbau. Die Tests wurden mit drei biologischen und drei technischen Wiederholungen durchgeführt. Die ermittelte antimikrobielle Aktivität ist ausgedrückt als Mittelwert der logarithmisch dargestellten Anzahl vermehrungsfähiger Bakterien im Vergleich zur Kontrolle. Die Auswertung der Ergebnisse zeigte eine Keimreduktion von $\geq 8$ log10 für P. aeruginosa und $\geq 6$ log10 für S. aureus. Die Ergebnisse sind dargestellt in Fig. 3a (*P. aeruginosa*) und Fig. 3b (*S. aureus*). Wie aus der Darstellung deutlich wird, setzte bereits nach Initialkontakt mit der Beschichtung (0 h) eine starke antimikrobielle Wirkung ein, deren Effekt nach 24 h noch ausgeprägter war. Die Befeuchtung der Vliesstoffe mit PBS hatte keinen Einfluss auf die Ergebnisse (nicht dargestellt).

**Beispiel 5b: Antimikrobielle Wirksamkeit einer polylysinhaltigen Tauchbeschichtung**

Zur Bestimmung der antimikrobiellen Aktivität von Vliesstoffen mit polylysinhaltigen Tauchbeschichtungen wurden zunächst Vliesstoffe mit folgenden Beschichtungszusammensetzungen hergestellt: 48 alternierende Schichten, wobei jede Schicht entweder aus Polylysin oder Hyaluronsäure bestand (entsprechend 24 Doppelschichten); eingesetzte Konzentrationen: 10 mg / ml Polylysin und 0,5 mg/ml Hyaluronsäure.

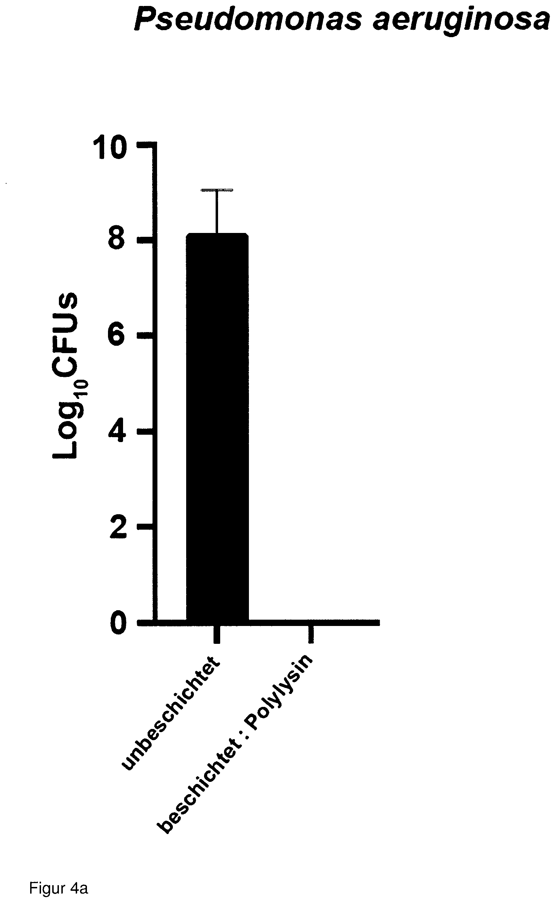

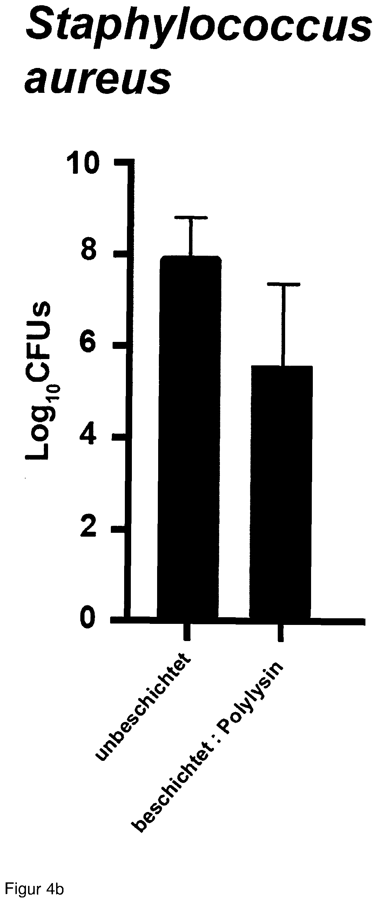

Die Beschichtung der Vliesstoffe erfolgte mittels des in Beispiel 2 beschriebenen Verfahrens, wobei lediglich die verwendeten Lösungen durch die in diesem Beispiel oben genannten Zusammensetzungen ersetzt wurden. Die antimikrobielle Wirksamkeit der Vliesstoffe mit polylysinhaltiger Tauchbeschichtung wurde wie unter Beispiel 5a angegeben getestet. Die Ergebnisse sind in Fig. 4a (*P. aeruginosa*) und Fig. 4b (*S. aureus*) dargestellt, wobei die angegebenen Werte nach einem Zeitraum von 24 h erhoben wurden.

**Beispiel 5c: Vergleich der antimikrobiellen Wirksamkeit von tauchbeschichteten Vliesstoffen mit Polyarginin, Polylysin und einer Mischung dieser beiden Polypeptide**

Zur Bestimmung der antimikrobiellen Aktivität von Vliesstoffen mit polyargininhaltigen sowie polylysinhaltigen Tauchbeschichtungen wurden Vliesstoffe mit folgenden Beschichtungszusammensetzungen in ihrer antimikrobiellen Wirksamkeit verglichen:

- 48 alternierende Schichten, wobei jede Schicht entweder aus Polyarginin oder Hyaluronsäure bestand (entsprechend 24 Doppelschichten); eingesetzte Konzentrationen: 0,5 mg / ml Polyarginin und 0,5 mg / ml Hyaluronsäure (Beschichtung gemäß Beispiel 5a)

- 48 alternierende Schichten, wobei jede Schicht entweder aus Polylysin oder Hyaluronsäure bestand (entsprechend 24 Doppelschichten); eingesetzte Konzentrationen: 10 mg/ml Polylysin und 0,5 mg/ml Hyaluronsäure (Beschichtung gemäß Beispiel 5b)

- 48 alternierende Schichten, wobei jede Schicht entweder aus einer Mischung von Polylysin mit Polyarginin oder aber aus Hyaluronsäure bestand (entsprechend 24 Doppelschichten); eingesetzte Konzentrationen: 10 mg/ml Polylysin, 0,5 mg /ml Polyarginin und 0,5 mg/ml Hyaluronsäure (Beschichtung der Vliesstoffe analog zu Beispiel 2).

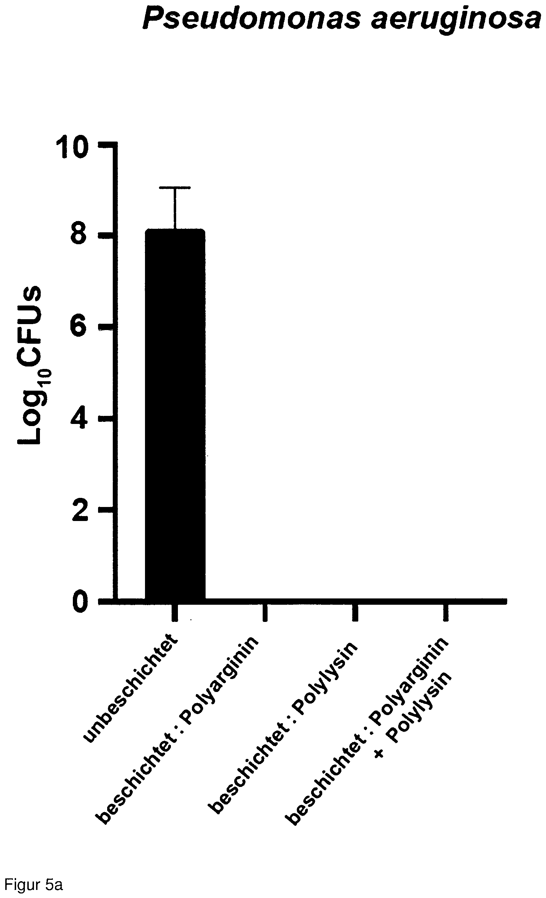

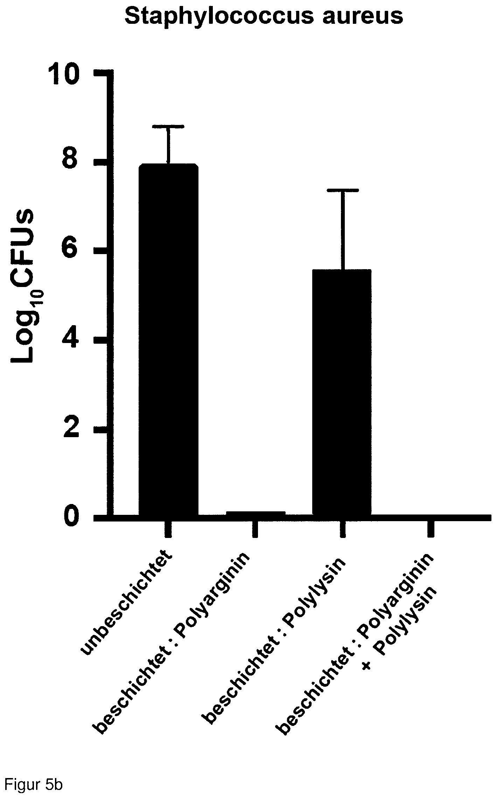

Die Beschichtung der Vliesstoffe erfolgte mittels des in Beispiel 2 beschriebenen Verfahrens, wobei lediglich die verwendeten Lösungen durch die in diesem Beispiel oben genannten Zusammensetzungen ersetzt wurden. Die antimikrobielle Wirksamkeit der Vliesstoffe wurde wie unter Beispiel 5a angegeben getestet. Die Tests erfolgten in dreifacher biologischer und dreifacher technischer Wiederholung. Die Mittelwerte der Ergebnisse sind in Fig. 5a (*P. aeruginosa*) und Fig. 5b (*S. aureus*) dargestellt, wobei die angegebenen Werte nach einem Zeitraum von 24 h erhoben wurden.

**Beispiel 6a: Antimikrobielle Wirksamkeit einer polylysinhaltigen Sprühbeschichtung**

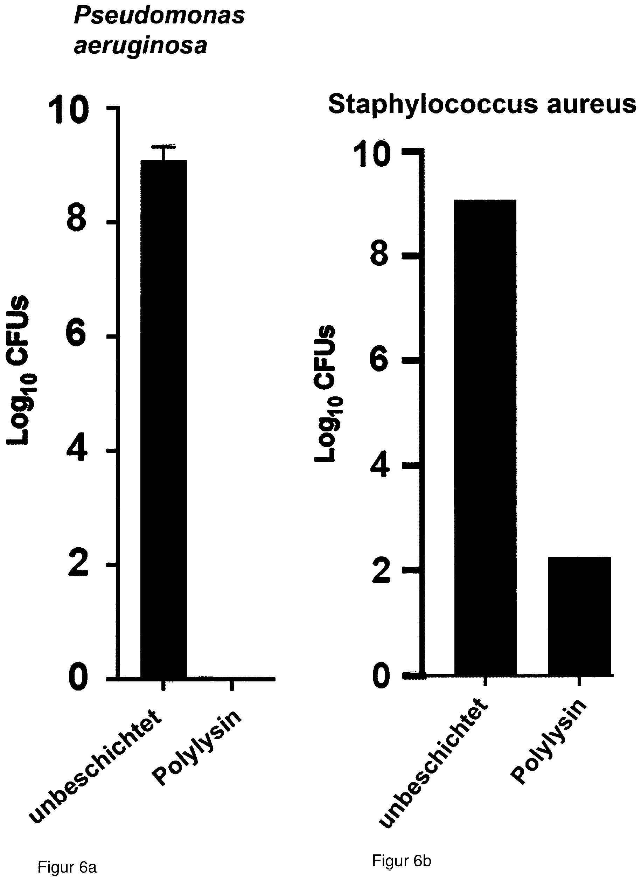

Für den Test eingesetzt wurden die in Beispiel 3a mittels Sprühverfahren mit einer Beschichtung versehenen Vliesstoffe. Die Vliesstoffe enthielten eine Sprühbeschichtung bestehend aus einer durchmischten Lage enthaltend ε-Poly(L-Lysin) (10 mg / ml) und HA144 (0.5mg / ml). Die antimikrobielle Wirksamkeit der mittels Spühbeschichtung ausgestatteten Vliesstoffe wurde mittels ISO 20743:2021 getestet. Die Messung wurde insgesamt drei Mal durchgeführt und die Mittelwerte gebildet. Weitere Details zum Ablauf dieses Tests können Beispiel 5 entnommen werden, wobei im vorliegenden Fall keine Messwerte für den Initialkontakt (t = 0) genommen wurden. Fig. 6a und Fig. 6b zeigen die nach 24 h erhobenen Messwerte. Wie aus den Darstellungen deutlich wird, war ein ausgeprägter antimikrobieller Effekt nachweisbar.
Die Auswertung der Ergebnisse zeigte eine Keimreduktion von $\geq$ 9 log10 für *P. aeruginosa* und $\geq$ 7 iog10 für *S. aureus*, was die bereits hohen Keimreduktionsraten der tauchbeschichteten Vliesstoffe nochmals überstieg.

**Beispiel 6b: Antimikrobielle Wirksamkeit einer polyargininhaltigen Sprühbeschichtung**

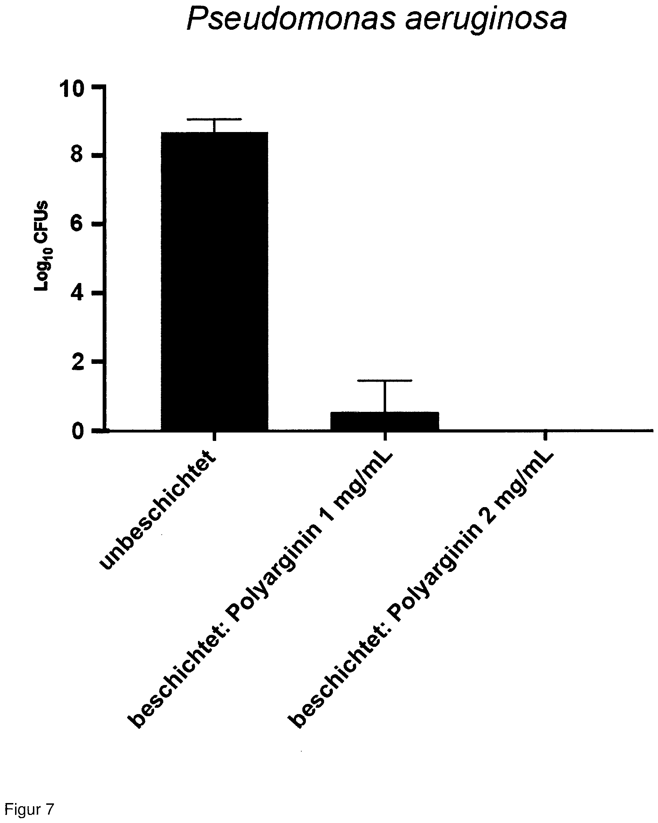

Für den Test eingesetzt wurden die in Beispiel 3b mittels Sprühverfahren erzeugten Vliesstoffe mit den beiden aufgebrachten Konzentrationen an Polyarginin. Das Vorgehen war analog zu Beispiel 6a, wobei in diesem Fall ausschließlich gegen *P. aeruginosa* getestet wurde. Die Ergebnisse sind in Fig. 7 dargestellt. Wie aus der Darstellung deutlich wird, war nach 24 h ein ausgeprägter antimikrobieller Effekt nachweisbar. Bei Einsatz von 2 mg/ml waren sogar keinerlei koloniebildenden Einheiten mehr nachweisbar.

**Beispiel 7: Vergleich der antimikrobiellen Wirksamkeit zwischen sprühbeschichtetem Vliesstoff und einer silberhaltigen Wundauflage**

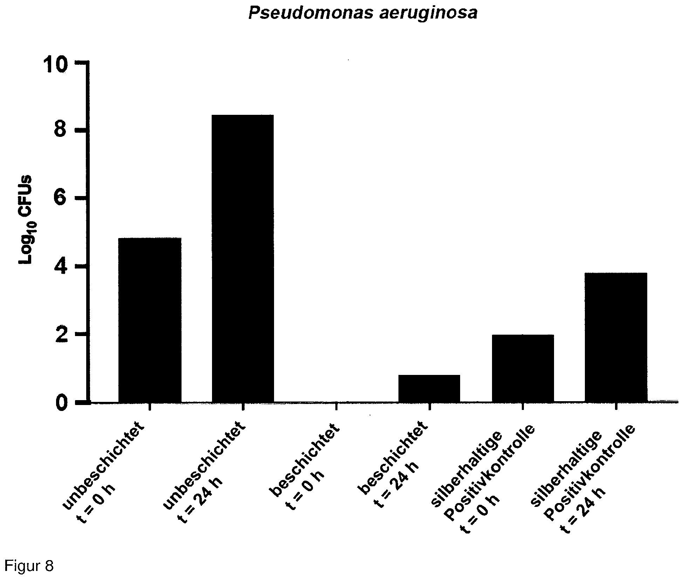

Die in Beispiel 6a hergestellten Vliesstoffe mit polylysinhaltiger Sprühbeschichtung wurden in ihrer antimikrobiellen Wirksamkeit verglichen mit einer im Handel verfügbaren Wundauflage mit ionischem Silber, EDTA und Benzethoniumchlorid. Die Messung der antimikrobiellen Wirkung erfolgte nach dem in Beispiel 5a beschriebenen Ansatz gegen *P. aeruginosa.* Die Anzahl der koloniebildenden Einheiten (CFU) wurden zu den beiden Zeitpunkten $T_0$ (nach Initialkontakt) und $T_{24h}$ (nach Ablauf von 24h) bestimmt. Als Negativkontrolle diente ein baugleicher Vliesstoff ohne Beschichtung.
Die Ergebnisse sind in Fig. 8 abgebildet, wobei es sich um den dekadischen Logarithmus der nachgewiesenen CFUs handelt. Wie der Abbildung entnommen werden kann, zeigte der silberhaltige Vergleichsverband ausgeprägte antimikrobielle Eigenschaften, die jedoch von den erfindungsgemäß beschichteten Vliesstoffen nochmals deutlich übertroffen wurden. So wurden nach Initialkontakt bei der Negativkontrolle etwa $10^5$ CFU nachgewiesen, bei der silberhaltigen Wundauflage etwa $10^2$ CFU und bei dem beschichteten Vliesstoff waren keine koloniebildenden Einheiten nachweisbar. Auch nach Ablauf von 24h zeigte der beschichtete Vliesstoff die höchste antimikrobielle Wirksamkeit mit einer (im Vergleich mit der Negativkontrolle) um mehr als sieben Logstufen reduzierten Koloniezahl.

**Beispiel 8: Trocknung von Vliesstoffen mit aufgesprühter Beschichtung**

Die in den oben beschriebenen Beispielen hergestellten beschichteten Vliesstoffe wurden nach dem Beschichtungsprozess über Nacht bei Raumtemperatur getrocknet.

Zusätzlich fanden antimikrobielle Versuche mit sprühbeschichteten, noch feuchten Proben sowie mit sprühbeschichteten Proben, die 10 min lang bei 80 °C getrocknet wurden, statt. Die Sprühbeschichtung wurde gemäß Beispiel 3 aufgetragen und enthielt ε-Poly(L-Lysin) sowie HA144. Die ermittelte antimikrobielle Wirkung war in allen Fällen nahezu identisch (Ergebnisse nicht dargestellt), so dass geschlussfolgert werden kann, dass die antiseptische Wirksamkeit auch bei unterschiedlichem Feuchtigkeitsgehalt der Beschichtung sowie nach aktiver Trocknung erhalten bleibt.

**Beispiel 9: Sterilisation beschichteter Wundkontaktschichten**

Die in Beispiel 2 mittels Tauchverfahren hergestellten beschichteten Vliesstoffe wurden bei 120 °C über einen Zeitraum von 20 Minuten autoklaviert. Die Muster zeigten nach Ablauf der Sterilisation keine erkennbaren Auffälligkeiten. Die anschließende Überprüfung der antimikrobiellen Aktivität (analog zu Beispiel 5a) ergab ebenfalls keine Unterschiede zum vorherigen Test (Ergebnisse nicht dargestellt).

**Beispiel 10: Wundauflagen enthaltend Vliesstoffe**

Bereitgestellt wurden Wundauflagen, die einen Vliesstoff als Wundkontaktschicht verwenden und sich mit der erfindungsgemäßen Beschichtung ausstatten lassen:

A: Wundauflage mit absorbierenden Zellstoffflocken

Bereitgestellt wurde eine Wundauflage in Form eines absorbierenden Kissens. Das absorbierende Kissen enthielt in proximaler Ausrichtung einen weißen Vliesstoff bestehend aus dem in Beispiel 1 angegebenen Vliesstoff B mit einer Fläche von 9 cm x 9 cm. In distaler Ausrichtung enthielt das absorbierende Kissen ein grünes Vliesmaterial aus reinem Polypropylen (Flächengewicht 45 g/m$^2$). Der proximal gelegene Vliesstoff und das distal gelegene Vliesmaterial hatten dieselbe Ausdehnung und waren lediglich entlang ihres Randes ultraschallverschweißt. Durch die am Rand befindliche Schweißnaht entstand auf diese Weise eine geschlossene Tasche, in deren Innerem sich ein Hohlraum befand. Dieser Hohlraum war ausgefüllt mit einem Tissue-Papier, in welchem absorbierende Zellstoffflocken eingewickelt waren. Das Tissue-Papier stellte dabei eine Verteilerschicht dar, die aufgenommene Flüssigkeit zumindest in lateraler (horizontaler) Richtung verteilen kann, aber auch in vertikaler Richtung durchlässig ist.

Der proximal gelegene, weiße Vliesstoff war geeignet, um mit der antimikrobiellen, erfindungsgemäßen Beschichtung versehen zu werden. Beim fertigen Produkt können Anwender anhand der unterschiedlichen Farben (grün und weiß) unterscheiden, welche Seite des Kissens auf die Wunde aufzulegen ist.

B: Wundauflage mit superabsorbierenden Partikeln

Bereitgestellt wurde eine Wundauflage in Form eines absorbierenden Kissens. Das absorbierende Kissen enthielt in proximaler Ausrichtung einen weißen Vliesstoff bestehend aus dem in Beispiel 1 angegebenen Vliesstoff A mit einer Fläche von 8,9 cm x 8,9 cm. In distaler Ausrichtung enthielt das absorbierende Kissen ein grünes Vliesmaterial aus reinem Polypropylen (Flächengewicht 25 g/m$^2$). Der proximal gelegene Vliesstoff und das distal gelegene Vliesmaterial hatten dieselbe Ausdehnung und waren lediglich entlang ihres Randes ultraschallverschweißt. Durch die am Rand befindliche Schweißnaht entstand auf diese Weise eine geschlossene Tasche, in deren Innerem sich ein Hohlraum befand. Dieser Hohlraum war ausgefüllt mit einem Tissue-Papier, in welchem absorbierende Zellstoffflocken und superabsorbierende Partikel aus Polyacrylsäure eingewickelt waren.

Der proximal gelegene, weiße Vliesstoff war geeignet, um mit der antimikrobiellen, erfindungsgemäßen Beschichtung versehen zu werden.

C: Wundauflage in Form eines Inselverbands

Bereitgestellt wurde eine Wundauflage, die baugleich mit der Wundauflage aus dem oberen Beispiel B war. Die Wundauflage wurde zusätzlich mit einer rückseitigen (distalen) Backingschicht in Form eines Films aus Polyurethan ausgestattet. Der Film war durchlässig für Luft und Wasserdampf, aber undurchlässig für Wasser. Die Befestigung erfolgte über einen Acrylkleber, der auf die proximale Seite des Polyurethanfilms aufgetragen war. Das besagte Backing hatte eine Fläche von 12,5 cm x 12,5 cm. Das Wundkissen wurde mittig an das Backing befestigt, so dass die Wundauflage über einen umlaufenden Kleberand verfügte (Inselform).

Der proximal gelegene Vliesstoff war geeignet, um mit der antimikrobiellen, erfindungsgemäßen Beschichtung versehen zu werden. Die Wundauflage kann nach erfolgter Beschichtung an ihrer Unterseite (proximale Ausrichtung) mit einer Schutzfolie aus Polyethylen versehen werden. Die Schutzfolie haftet am umlaufenden Kleberand und wird unmittelbar vor dem Anbringen der Wundauflage vom Anwender entfernt.

**Beispiel 11: Simultane Beschichtung mehrerer Vliesstoffe mittels automatisiertem Roboter und Bestimmung der antimikrobiellen Wirksamkeit**

Es wurden acht Vliesstoffe vom Typ A zeitgleich im Tauchverfahren mittels eines programmierbaren Roboters mit 48 Einzellagen (24 Doppellagen) aus jeweils Hyaluronsäure oder Polyarginin (Materialien gemäß Beispiel 1) beschichtet. Der Ablauf erfolgte analog zu Beispiel 5a.

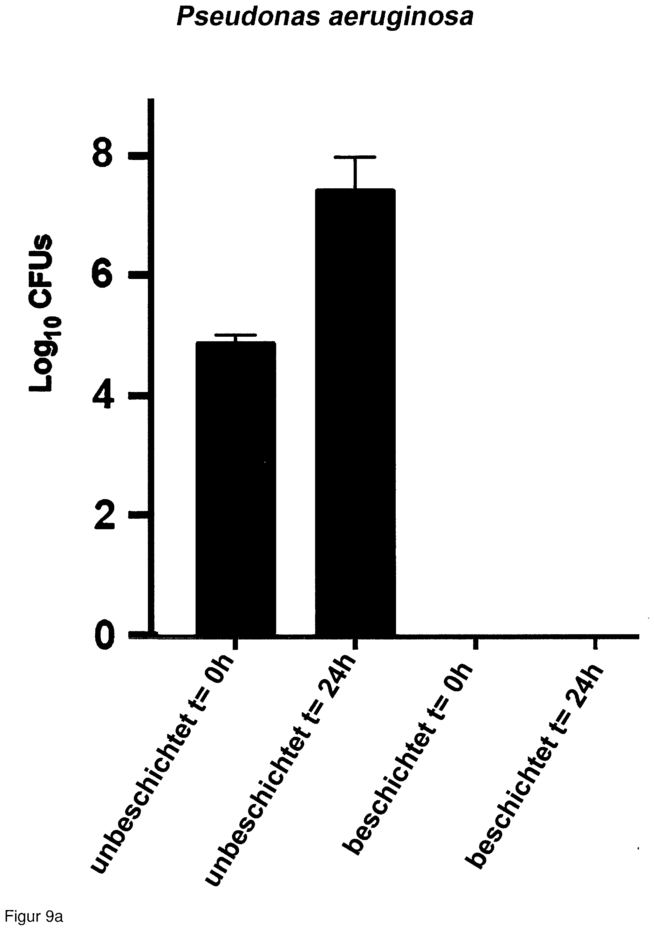

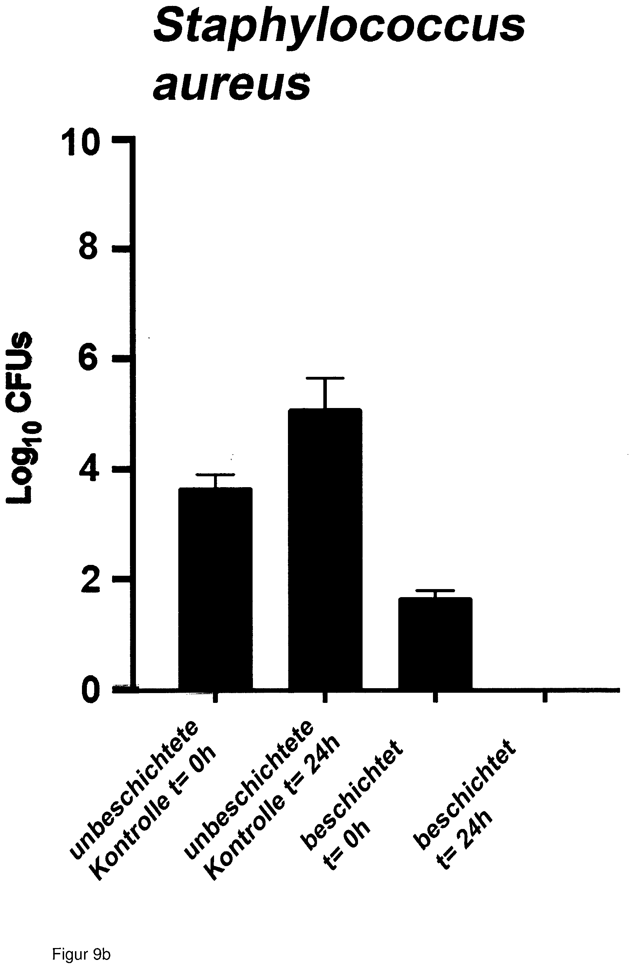

Die derart erzeugten Vliesstoffe wurden anschließend auf ihre antimikrobielle Wirksamkeit getestet. Der Test erfolgte analog zu Beispiel 5a. Die Ergebnisse sind in Fig. 9a für *Pseudomonas aeruginosa* und in Fig. 9b für *Staphylococcus aureus* dargestellt.

Wie aus den Ergebnissen ersichtlich wird, hatten auch die simultan hergestellten Vliesstoffe eine hohe antimikrobielle Wirksamkeit. So waren koloniebildende Einheiten von *P. aeruginosa* bereits nach Initialkontakt nicht mehr nachweisbar. *S. aureus* war nach 24h nicht mehr nachweisbar. Im Gegensatz dazu war bei der Negativkontrolle sowohl für *P. aeruginosa* als auch für *S. aureus* ein deutlicher Anstieg der koloniebildenden Einheiten nach 24h zu verzeichnen.

**Beispiel 12: Ausstattung einer Wundauflage mit einem beschichteten Vliesstoff**

Bereitgestellt wurde ein Vliesstoff vom Typ A mit einer Fläche von 2,5 cm x 2,5 cm. Die Beschichtung fand gemäß Beispiel 2 statt. Anschließend wurde der beschichtete Vliesstoff als Wundkontaktschicht in einer Wundauflage eingesetzt, so dass die Beschichtung nach außen zeigte. Der Aufbau der Wundauflage kann - mit Ausnahme der Maße - Beispiel 10b entnommen werden. Erhalten wurde eine atraumatische Wundauflage mit sehr guten Absorptionseigenschaften und einer antimikrobiellen Beschichtung.

**Patentansprüche**

1. Vliesstoff zur Wundbehandlung, welcher teilweise oder vollständig eine antimikrobielle Beschichtung aufweist, welche i) eine Hyaluronsäure sowie ii) ein Polypeptid ausgewählt aus Polyarginin, Polylysin und Polyornithin oder eine Mischung aus mindestens zwei der zuvor genannten Polypeptide umfasst.

2. Vliesstoff nach Anspruch 1, wobei der Vliesstoff mindestens eines der folgenden Materialien oder Materialgemische enthält: Kunstfasern, Polyolefin basierte Fasern, Polyethylen, Polypropylen, Polyamid, Viskose, Zellulose basierte Fasern, eine Mischung aus Polyamid und Viskose, eine Mischung aus Polypropylen und Viskose.

3. Vliesstoff nach Anspruch 1 oder 2, wobei der Vliesstoff mindestens 50 Gew.-% Polypropylen und / oder mindestens 20 Gew.-% Viskose enthält.

4. Vliesstoff nach einem der vorhergehenden Ansprüche enthaltend Kunstfasern und / oder hydrophobe Fasern, die durch eine chemische oder physikalische Behandlung hydrophiliert wurden.

5. Vliesstoff nach einem der vorhergehenden Ansprüche, wobei der Vliesstoff mindestens zwei miteinander verbundene Vliesschichten unterschiedlicher Zusammensetzung umfasst, wobei die wundzugewandte Vliesschicht Kunstfasern enthält und die antimikrobielle Beschichtung aufweist und wobei die wundabgewandte Vliesschicht Viskose enthält und dadurch die laterale Verteilung absorbierter Wundflüssigkeit vermittelt.

6. Vliesstoff nach einem der vorhergehenden Ansprüche, wobei der Vliesstoff ein Flächengewicht von 20 g/m$^2$ bis 100 g/m$^2$ hat.

7. Vliesstoff nach einem der vorhergehenden Ansprüche, wobei das Polypeptid mindestens zehn Aminosäuren und/oder höchstens hundert Aminosäuren umfasst.

8. Vliesstoff nach einem der vorhergehenden Ansprüche, wobei die Hyaluronsäure als Polymermischung mit unterschiedlicher Kettenlänge vorliegt und wobei die Polymermischung Polymere der Hyaluronsäure mit einem Molekulargewicht von mindestens 10 kDa und / oder höchstens 300 kDa umfasst.

9. Vliesstoff nach einem der vorhergehenden Ansprüche, wobei die Beschichtung mindestens zwei übereinanderliegende und miteinander verbundenen Lagen enthält, und wobei mindestens eine Lage vorhanden ist, welche das Polypeptid enthält, und eine positive Nettoladung hat, sowie mindestens eine Lage, welche die Hyaluronsäure enthält und eine negative Nettoladung hat, und wobei sich bei den übereinanderliegenden Lagen jeweils eine das Polypeptid enthaltende Lage mit einer die Hyaluronsäure enthaltenden Lage abwechselt, so dass eine Abfolge von alternierenden Lagen gebildet wird.

10. Vliesstoff nach Anspruch 9, wobei die Anzahl der übereinanderliegenden und miteinander verbundenen Lagen in der Beschichtung 10 bis 100 beträgt.

11. Vliesstoff nach einem Ansprüche 1 bis 8, wobei das Polypeptid und die Hyaluronsäure innerhalb der Beschichtung durchmischt vorliegen und das Polypeptid in einer Hyaluronsäurematrix eingebettet ist.

12. Vliesstoff nach einem der vorhergehenden Ansprüche, wobei das Polypeptid ein Molekulargewicht von 1 bis 41 kDa hat.

13. Vliesstoff nach einem der vorhergehenden Ansprüche, wobei die Beschichtung eine Dicke von 10 nm bis 1000 nm hat.

14. Vliesstoff nach einem der vorhergehenden Ansprüche, wobei die Beschichtung trocknungsbeständig ist.

15. Wundauflage enthaltend den Vliesstoff nach einem der vorhergehenden Ansprüche als Wundkontaktschicht, wobei der Vliesstoff mindestens einen Teil einer proximalen Seite eines in der Wundauflage enthaltenen Wundkissens ausbildet, das ein hydrophiles, absorbierendes Material enthält.

16. Wundauflage nach Anspruch 15, wobei das hydrophile, absorbierende Material eine superabsorbierende Substanz umfasst.

17. Wundauflage nach Anspruch 15 oder 16, wobei die Wundauflage über ein Backing als distale Abschlussschicht verfügt, dessen proximale Seite mit einer adhäsiven Beschichtung ausgestattet ist und wobei die Flächenausdehnung des Backings andere Bestandteile der Wundauflage überragt, so dass ein umlaufender Kleberand ausgebildet wird, der während des Gebrauchs eine Anhaftung der Wundauflage ermöglicht.

18. Verfahren zur Herstellung eines Vliesstoffes nach einem der Ansprüche 11 bis 14, welcher eine antimikrobielle Beschichtung enthält, wobei das Verfahren die folgenden Schritte umfasst:

   i) Bereitstellung eines Vliesstoffs
   ii) Besprühen des Vliesstoffs mit einer Hyaluronsäure und einem Polypeptid zur Ausbildung einer Beschichtung umfassend mindestens eine Beschichtungslage, wobei das Polypeptid ausgewählt ist aus Polyarginin, Polylysin und Polyornithin oder einer Mischung aus mindestens zwei der zuvor genannten Polypeptide und wobei die mindestens eine Beschichtungslage sowohl die Hyaluronsäure als auch das Polypeptid enthält.

100 µm

Figur 1

Figur 2

**Pseudomonas aeruginosa**

Figur 3a

**Staphylococcus aureus**

Figur 3b

# *Pseudomonas aeruginosa*

Figur 4a

# Staphylococcus aureus

Figur 4b

# *Pseudomonas aeruginosa*

Figur 5a

# Staphylococcus aureus

Figur 5b

## Pseudomonas aeruginosa

Figur 6a

## Staphylococcus aureus

Figur 6b

Figur 7

Pseudomonas aeruginosa

Figur 8

# *Pseudonas aeruginosa*

Figur 9a

# *Staphylococcus aureus*

Figur 9b

Europäisches
Patentamt

European
Patent Office

Office européen
des brevets

## EUROPÄISCHER RECHERCHENBERICHT

Nummer der Anmeldung

EP 24 31 5419

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2022/211914 A1 (LAVALLE PHILIPPE [FR] ET AL) 7. Juli 2022 (2022-07-07) | 1-4,7,8, 11,12,14 | INV. A61L15/22 |
| Y | * Seite 2, Absatz 30 *<br>* Seite 8, Absatz 161 *<br>* Seite 12, Absatz 252 *<br>* Beispiel 5 *<br>* Ansprüche 1, 2 * | 5,6,9, 10,13, 15-18 | A61L15/46 A61L15/60 |
| | ----- | | |
| A | EP 1 120 428 A2 (KURARAY CO [JP]) 1. August 2001 (2001-08-01)<br>* Seite 5, Absatz 39 *<br>* Seite 7, Absatz 68 *<br>* Ansprüche 1, 8, 9 * | 1-15 | |
| | ----- | | |
| Y | EP 0 047 492 A2 (INTERMEDICAT GMBH [CH]) 17. März 1982 (1982-03-17)<br><br>* Anspruch 1 * | 5,6,9, 10,13, 15-18 | |
| | ----- | | |

RECHERCHIERTE SACHGEBIETE (IPC)

A61L

Der vorliegende Recherchenbericht wurde für alle Patentansprüche erstellt

| Recherchenort | Abschlußdatum der Recherche | Prüfer |
|---|---|---|
| Den Haag | 18. Februar 2025 | Heck, Georg |

KATEGORIE DER GENANNTEN DOKUMENTE

X : von besonderer Bedeutung allein betrachtet
Y : von besonderer Bedeutung in Verbindung mit einer
  anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
  nach dem Anmeldedatum veröffentlicht worden ist
D : in der Anmeldung angeführtes Dokument
L : aus anderen Gründen angeführtes Dokument
...............................................................................................
& : Mitglied der gleichen Patentfamilie, übereinstimmendes
  Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 24 31 5419

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-02-2025

| Im Recherchenbericht angeführtes Patentdokument | Datum der Veröffentlichung | Mitglied(er) der Patentfamilie | Datum der Veröffentlichung |
|---|---|---|---|
| US 2022211914 A1 | 07-07-2022 | CA 3135244 A1 | 05-11-2020 |
| | | CN 114040785 A | 11-02-2022 |
| | | EP 3797800 A1 | 31-03-2021 |
| | | EP 3962543 A1 | 09-03-2022 |
| | | ES 2981338 T3 | 08-10-2024 |
| | | JP 7587524 B2 | 20-11-2024 |
| | | JP 2022531769 A | 11-07-2022 |
| | | US 2022211914 A1 | 07-07-2022 |
| | | WO 2020221896 A1 | 05-11-2020 |
| EP 1120428 A2 | 01-08-2001 | DE 60118637 T2 | 11-01-2007 |
| | | EP 1120428 A2 | 01-08-2001 |
| | | ES 2260098 T3 | 01-11-2006 |
| | | KR 20010076418 A | 11-08-2001 |
| | | US 2001018464 A1 | 30-08-2001 |
| EP 0047492 A2 | 17-03-1982 | DE 3033606 A1 | 08-04-1982 |
| | | EP 0047492 A2 | 17-03-1982 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1755569 B9
- EP 3452118 B1
- EP 2371335 B1
- WO 2010131175 A1

**In der Beschreibung aufgeführte Nicht-Patentliteratur**

- *Medical mycology*, 2017, vol. 55 (3), 334-343
- *Interdisciplinary Toxicology*, 2015, vol. 8 (4), 193-202
- *J Appl Microbiol.*, 2021, vol. 130 (4), 1012-22